# EUROPÄISCHE PATENTSCHRIFT

(11) **EP 3 990 916 B1**
(45) Veröffentlichungstag und Bekanntmachung des Hinweises auf die Patenterteilung: **07.06.2023**
(21) Anmeldenummer: 20734914.3
(22) Anmeldetag: 25.06.2020
(51) Int. Cl.: G01N 33/22

(54) **VERFAHREN ZUR BRENNWERTBESTIMMUNG, VERFAHREN ZUR ENERGIEMENGENBESTIMMUNG EINES GASES UND VORRICHTUNG HIERZU**
METHOD FOR DETERMINING A CALORIFIC VALUE, METHOD FOR DETERMINING THE QUANTITY OF ENERGY OF A GAS AND DEVICE FOR THIS PURPOSE
PROCÉDÉ DE DÉTERMINATION DE POUVOIR CALORIFIQUE, PROCÉDÉ DE DÉTERMINATION DE QUANTITÉ D'ÉNERGIE D'UN GAZ ET DISPOSITIF CORRESPONDANT

(30) Priorität: 27.06.2019 DE 102019117427
(43) Veröffentlichungstag der Anmeldung: 04.05.2022
(73) Patentinhaber: EnBW Energie Baden-Württemberg AG, 76131 Karlsruhe (DE)
(72) Erfinder: KESSLER, Alois, 75417 Mühlacker (DE); BREMER, Antje, 76228 Karlsruhe (DE)
(74) Vertreter: RPK Patentanwälte Reinhardt und Kaufmann Partnerschaft mbB
(86) Internationale Anmeldenummer: PCT/EP2020/067824
(87) Internationale Veröffentlichungsnummer: WO 2020/260461

(56) Entgegenhaltungen:
- EP-A1- 1 193 488
- EP-A1- 2 963 413
- EP-A2- 0 959 354
- WO-A1-2011/096799
- CN-A- 108 831 121
- RU-C1- 2 586 446
- US-A- 4 542 640
- US-A1- 2017 101 947
- US-A1- 2017 370 831
- KOENIG R: "ZUR BESCHR[NKUNG DES ANSPRUCHSINHALTS DURCH"BAR"-DERIVATE", MITTEILUNGEN DER DEUTSCHEN PATENTANWAELTE, HEYMANN, KOLN, DE, 1 January 1997 (1997-01-01), page 62, XP001248632, ISSN: 0026-6884
- WU PAN ET AL: "Application of electronic nose in gas mixture quantitative detection", NETWORK INFRASTRUCTURE AND DIGITAL CONTENT, 2009. IC-NIDC 2009. IEEE INTERNATIONAL CONFERENCE ON, IEEE, PISCATAWAY, NJ, USA, 6 November 2009 (2009-11-06), pages 976-980, XP031585955, ISBN: 978-1-4244-4898-2

## Beschreibung

### Stand der Technik

Die Erfindung betrifft ein Verfahren zur Brennwertbestimmung eines Gases, ein Verfahren zur Energiemengenbestimmung sowie eine Vorrichtung hierzu.

Gasnetze sind vermaschte Systeme, typischerweise mit mehreren Ein- und Ausspeisestellen, an denen Erdgas eingespeist wird, bzw. Erdgas entnommen wird, um Verbraucher zu versorgen. Es wird zwischen Hochdruck-, Mitteldruck- und Niederdruck-Leitungen unterschieden. Die Druckstufen entsprechen weitgehend dem Zweck als Transportnetz, Verteilnetz und Ortsnetz. Die genaue Zusammensetzung des Erdgases unterscheidet sich je nach Herkunft. Grob wird zwischen hochkalorischem Gas ("H-Gas") und niederkalorischem Gas ("L-Gas") unterschieden. H-Gas kommt beispielsweise aus Russland und hat mit einem Methangehalt von etwa 95% einen deutlich höheren Energiegehalt als sogenanntes L-Gas beispielsweise aus den Niederlanden.

Üblicherweise wird bei der Verteilung und Abrechnung von Erdgas über das Gasnetz von quasi-stationären Bedingungen ausgegangen. Nach dem derzeit gültigen deutschen Eichgesetz ist der Brennwert beim Endkunden mit einer maximalen Unsicherheit von 2% entsprechend der Verkehrsfehlergrenze zu bestimmen. Alle bisher bekannten Verfahren und Regelwerke zielen deshalb auf eine möglichst konstant eingestellte Gasbeschaffenheit ab.

Durch Zudosierung beispielsweise von Propan zur Erhöhung oder Stickstoff zur Erniedrigung des Brennwerts wird eine je nach Gasherkunft und Mischung unterschiedliche Gasbeschaffenheit ausgeglichen und somit ein stabiler Brennwert eingestellt, auf dessen Basis später die monatliche Abrechnung erfolgt.

Die Gasabrechnung ist in der Technischen Regel DVGW G685:2008 von 2008 des deutschen Vereins des Gas- und Wasserfaches e.V. vorgegeben. Die Abrechnung des gelieferten Gases erfolgt üblicherweise als Energiemenge (kWh bzw. MWh). Dafür wird zunächst der volumetrische Brennwert mittels eines Prozessgaschromatographen ("PGC") oder Kalorimeters an etwa 500 Stellen im deutschen Erdgasnetz ermittelt. Das lokal beim Kunden mittels Gaszähler gemessene Gasvolumen wird an ein Abrechnungssystem übermittelt. Für die Abrechnung der Brennwerte werden von einem sogenannten Rekonstruktionssystem mittlere monatliche Brennwerte für einzelne Gasnetzabschnitte (sog. Brennwert-Bezirke) gebildet und an das Abrechnungssystem übermittelt. Aus dem Produkt von Brennwert und Abrechnungsvolumen wird schließlich die bezogene Energiemenge errechnet.

Die Anforderungen an die Gasbeschaffenheit sind im DVGW-Regelwerk definiert. Wasserstoff darf nur bis 2 vol.% im Erdgas enthalten sein, wenn sensible Anwendungen wie etwa Tankstellen für Erdgasfahrzeuge angeschlossen sind, ansonsten ist ein Anteil von bis zu 10 vol.% zulässig.

Im Zuge der Energiewende wird zunehmend auch über eine Reduktion der CO₂-Emissionen des Gassektors diskutiert. Dies kann durch das Beimischen von so genanntem Biomethan aus Biogasanlagen und/oder Wasserstoff aus CO₂-armer Herstellung in das Erdgasnetz erreicht werden. Derzeit wird bereits über Anpassungen des Regelwerks zur Gasbeschaffenheit diskutiert, um die Einspeisung relevanter Mengen an Wasserstoff zu ermöglichen. Durch die unterschiedlichen Eigenschaften, vor allem unterschiedliche Brennwerte, von Erdgas bzw. (Bio-)Methan einerseits und insbesondere Wasserstoff andererseits ergeben sich neue Anforderungen an Messung und Abrechnung des an Kunden gelieferten Gasgemisches. Dies ist insbesondere dann der Fall, wenn die Erzeugung von CO₂-armem Wasserstoff an die Verfügbarkeit von erneuerbarem Strom geknüpft ist und somit dessen Einspeisung in das Erdgasnetz einer hohen Volatilität unterliegen kann.

Erfolgt die volatile Einspeisung relevanter Mengen an Wasserstoff im zweistelligen Prozentbereich ergeben sich vollkommen neue Anforderungen an die Messung und Abrechnung des Gases. Das bisherige Verfahren der zentrale Brennwert-Bestimmung und Brennwert-Einstellung und die zusätzliche lokale Volumenmessung zur Bildung der Abrechnungswerte wird angesichts dieser Veränderungen im Erdgassystem alsbald an Grenzen stoßen.

Aus der RU 2586446 C1 ist ein Verfahren zur Analyse einer Gaszusammensetzung der Umgebung durch Messen der Impedanz einer gasempfindlichen Halbleiterschicht, eines segmentierten Satzes koplanarer Elektroden in einem Multi-Chip bekannt. Ein Merkmal des Verfahrens ist die Verwendung einer Niederfrequenz (10⁻² -10⁶ Hz), bei der eine, durch Adsorption von Gasen verursachte, Impedanz Änderung langsame Ladungstransportprozesse in gasempfindlichem Halbleitermaterial ermöglicht, die eine entsprechende Änderung der, in dem Verfahren zur Problemlösung verwendeten, Ersatzschaltbildelemente definieren die Analyse der Gaszusammensetzung. Die Messung einer größeren Anzahl von Sensorchipsegmenten kann die Dimension der analysierten Vektorsignale erhöhen und die Genauigkeit der Identifizierung von Gas erhöhen.

Aus der CN 108831121 A ist ein Frühwarnverfahren und eine Vorwarnvorrichtung zur Herstellung von Minensicherheit bekannt. Das Verfahren umfasst einen Schritt zum Sammeln eines von jedem Gassensor ausgegebenen Widerstandswerts in einer Gassensoranordnung. Die Gassensoranordnung umfasst einen Methansensor, einen Kohlenmonoxidsensor, einen Wasserstoffsensor und einen Sauerstoffsensor. Das Verfahren umfasst weiter einen Schritt zum Bestimmen eines Methankonzentrationswert, eines Kohlenmonoxidkonzentrationswert, eines Wasserstoffkonzentrationswert und eines Sauerstoffkonzentrationswert gemäß den von den Gassensoren ausgegebenen Widerstandswerten. Das Verfahren umfasst weiter einen Schritt des Vergleichens eines Verhältnisses des Methankonzentrationswerts zum Kohlenmonoxidkonzentrationswert zum Wasserstoffkonzentrationwert und auf den Sauerstoffkonzentrationswert mit einem voreingestellten Explosionsverhältnis und Vergleichen des Methankonzentrationswerts und des Kohlenmonoxidkonzentrationswerts mit entsprechenden voreingestellten Vergiftungskonzentrationswerten, um Vergleichsergebnisse zu erhalten. Das Verfahren umfasst einen Schritt des Bestimmens eines Warnungstyps gemäß dem Vergleichsergebnis und des Aussendens einer entsprechenden Warnung.

Aus der US 4 542 640 A ist ein System zum qualitativen und quantitativen Nachweis und zur Identifizierung von Gasen mit Halbleitergassensoren bekannt. Das System umfasst Halbleitersensoren, welche auf brennbare Gase mit Erkennungsschwellen für Wasser-stoff, Kohlenmonoxid und Ethanolgase im ppm-Bereich reagieren. Diese Gassensoren adsorbieren reduzierende oder brennbare Gase wie Kohlenwasserstoffe, Wasserstoff, Kohlenmonoxid, Wasser, Methan, Alkohole, Rauch und andere und wandeln die Konzentrationen der Gase durch Änderung des Widerstandes in ein elektrisches Signal um. Das System umfasst eine Anzahl der einzelnen Sensoren, die größer oder gleich der Anzahl der ausgewählten zu unterscheidenden Gase ist. Jeder Sensor des Systems weist eine Reaktionscharakteristik auf, die sich von den Reak-tionseigenschaften der anderen Sensoren im Array in ihrer Reaktion auf mindestens eines der ausgewählten Gase unterscheidet. Vor der Verwendung im Detektionssystem werden die Widerstandsreaktionen jedes Sensors des Arrays auf jedes der ausgewählten Gase und auf Kombinationen der ausgewählten Gase gemessen. Diese Messungen werden verwendet, um Werte von Konstanten in spezifischen Gleichungen zu bestimmen, von denen jede in Form einer allgemeinen Antwortgleichung vorliegt. Ein elektrischer Prozessor führt durch analoge oder digitale Verarbeitung oder eine Kombination von jedem Mustererkennungslogikoperationen durch, die in der Lage sind, die Gasbestandteile, auf die das Array trifft, sofort anzuzeigen. Die Mustererkennungslogikoperationen werden von einem digitalen Prozessor ausgeführt, der mathematische Operationen zum Verarbeiten der Sensorsignale ausführt. Dies geschieht durch Lösen eines bestimmten Gleichungssystems, einer Gleichung für jeden Sensor des Arrays.

Aus der EP 1193488 A1 sind ein Verfahren und eine Vorrichtung zum Ermitteln der Gasbeschaffenheit eines Brenngases bekannt. Unter Gasbeschaffenheit wird die Gaszusammensetzung, der Brennwert, die Wobbezahl bzw. der Wobbeindex, die Normdichte und die Methanzahl verstanden. Hierbei wird ein Teil des Brenngases einer Infrarotstrahlung ausgesetzt. Für zwei Wellenlängen oder spektralen Bereiche wird der vom Brenngas absorbierte Anteil der Infrarotstrahlung gemessen. Zudem wird die Wärmeleitfähigkeit gemessen. Aus diesen drei Messwerten wird die Gasbeschaffenheit berechnet.

### Offenbarung der Erfindung

Die Aufgabe der Erfindung ist, ein Verfahren anzugeben, mit dem ein Brennwert bei volatiler Brenngaszusammensetzung zuverlässig bestimmt werden kann.

Eine weitere Aufgabe ist die Schaffung eines Verfahrens, mit dem eine Energiemenge bei volatiler Brenngaszusammensetzung zuverlässig bestimmt werden kann.

Eine weitere Aufgabe ist die Schaffung einer geeigneten Vorrichtung zur Bestimmung des Brennwerts und/oder der Energiemenge.

Die Aufgaben werden durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird nach einem ersten Aspekt der Erfindung ein Verfahren zur Bestimmung eines Brennwerts eines methanhaltigen Brenngases vorgeschlagen, bei dem das Brenngas ein oder mehrere Gasbestandteile in seiner Gaszusammensetzung aufweist. Das Verfahren umfasst die Schritte
(i) Beaufschlagen einer Sensoreinheit, die eine Mehrzahl von Teilsensoren umfasst, mit dem Brenngas, wobei unterschiedliche Teilsensoren unterschiedlich sensitiv auf denselben Gasbestandteil reagieren und/oder derselbe Teilsensor auf unterschiedliche Konzentrationen eines jeweils gleichen Gasbestandteils in der Gaszusammensetzung reagiert; wobei die Teilsensoren als unterschiedlich zusammengesetzte und/oder dotierte Halbleitersensoren ausgeführt sind, die durch UV-Bestrahlung und/oder IR-Bestrahlung und/oder eine Mikroheizung unterschiedlich aktiviert werden und durch die Anwesenheit reduzierender oder oxidierender Gasbestandteile eine messbare Widerstandsänderung erfahren,
(ii) Erfassen einer jeweiligen Widerstandsänderung als Sensorsignal, das von der Gaszusammensetzung und/oder der Konzentration von einem oder mehreren Gasbestandteilen abhängig ist;
(iii) Generieren eines Ist-Gasbeschaffenheitsmusters, das jeden Teilsensor und dessen jeweiliges Sensorsignal repräsentiert;
(iv) Bestimmen eines nächstkommenden Soll-Gasbeschaffenheitsmusters aus einer Gruppe von Soll-Gasbeschaffenheitsmustern;
(v) Extrahieren der Gaszusammensetzung und/oder der Konzentration von einem oder mehreren Gasbestandteilen aus dem nächstkommenden Soll-Gasbeschaffenheitsmuster oder mittels Interpolation aus mehreren Soll-Gasbeschaffenheitsmustern;
(vi) Bestimmen des aktuellen Brennwerts des Brenngases aus dem nächstkommenden Soll- Gasbeschaffenheitsmuster statt durch analytische Berechnung durch Vergleich des durch die Sensoreinheit gelieferten Ist-Gasbeschaffenheitsmusters mit dem nächstkommenden Soll-Gasbeschaffenheitsmuster oder mittels Interpolation aus mehreren Soll-Gasbeschaffenheitsmustern.

Sofern es kein eindeutiges nächstkommendes Soll-Gasbeschaffenheitsmuster gibt und mehrere Soll-Gasbeschaffenheitsmuster jeweils als nächstkommende Soll-Gasbeschaffenheitsmuster in Frage kommen, kann die Gaszusammensetzung und/oder Konzentration von Gasbestandteilen zwischen mehreren benachbarten Soll-Gasbeschaffenheitsmustern interpoliert werden.

Ebenso kann, sofern es kein eindeutiges nächstkommendes Soll-Gasbeschaffenheitsmuster gibt und mehrere Soll-Gasbeschaffenheitsmuster jeweils als nächstkommende Soll-Gasbeschaffenheitsmuster in Frage kommen, der Brennwert zwischen mehreren derartigen benachbarten Soll-Gasbeschaffenheitsmustern interpoliert werden.

Die Messung der Gasbeschaffenheit und die Bestimmung des Brennwerts, insbesondere eines Abrechnungsbrennwertes, gelingt somit mit einem einfachen Messverfahren, welches weitestgehend miniaturisiert ist und vollständig in einen lokalen Gaszähler integriert oder separat vorgesehen sein kann. Besonders vorteilhaft kann eine Sensoreinheit eingesetzt werden, deren Sensorik auf Halbleitersensoren beruht.

Bisher wird die Brennwertbestimmung mittels Prozessgaschromatographen (PGC) durchgeführt. Diese nutzen Wasserstoff als Trägergas. Der Wasserstoffanteil im Gas kann deshalb mit den üblichen Prozessgaschromatographen nicht ermittelt werden. In der Zukunft ist daher ein hoher Anpassungsaufwand durch Nachrüsten oder ersatzweisem Nutzen von modifizierten (He-) oder (Ar-)PGCs zu erwarten. Durch die beschriebene Erfindung kann dies langfristig entfallen, da mit dem erfindungsgemäßen Verfahren Wasserstoff im Gas erfasst werden kann.

Nach einer günstigen Ausgestaltung kann das Soll-Gasbeschaffenheitsmuster mittels mathematischer Methoden generiert werden. Beispielsweise kann das Generieren des Soll-Gasbeschaffenheitsmusters mittels neuronaler Netze, erfolgen. Alternativ oder zusätzlich kann das Ist-Gasbeschaffenheitsmuster mittels mathematischer Methoden, insbesondere mittels neuronaler Netze, analysiert werden.

Nach einer günstigen Ausgestaltung können die Soll-Gasbeschaffenheitsmuster generiert werden, indem die Sensoreinheit mit Referenzgasen mit einer vorgegebenen Konzentration an ein oder mehreren Gasbestandteilen beaufschlagt wird und den erhaltenen Sensorsignalen als Soll-Gasbeschaffenheitsmuster ein jeweils bekannter Brennwert des Referenzgases zugeordnet wird.

Es können in der Lernphase gezielt definierte Gase und/oder definierte Gaszusammensetzungen als Referenzgase mit unterschiedlichen Konzentrationen verschiedener Gasbestandteile bereitgestellt werden. Beim Training der Sensoreinheit mittels neuronalem Netz können die einzelnen Gasbestandteile jeweils schrittweise variiert werden. Die Sensorsignale der Teilsensoren der Sensoreinheit werden jeweils erfasst und so eine Auswerteinstanz trainiert. Dabei werden Soll-Gasbeschaffenheitsmuster erzeugt, deren Zusammensetzung genau bekannt ist. Dies erlaubt die sichere Erkennung auch komplexer Gaszusammensetzungen, wenn Ist-Gasbeschaffenheitsmuster mit Soll-Gasbeschaffenheitsmustern verglichen werden.

Ebenso können höhere Kohlenwasserstoffe wie Hexan, Heptan, Oktan, zyklische Kohlenwasserstoffe wie Benzol, Toluol oder Xylol, die bei verschiedenen Gasgemischen als charakteristische "Spurengase" enthalten sein können, für die eichrechtskonforme Bestimmung des Brennwerts mit genutzt werden, obwohl sie zum eigentlichen Energiegehalt des Gases keinen nennenswerten Beitrag leisten, wohl aber der "Erkennbarkeit" des Gases dienen können und gewissermaßen als "Fingerabdruck" oder als ein unverwechselbarer "Geruch" wahrgenommen werden. Die Methanmenge selbst als Hauptbestandteil von Erdgas wird durch die Halbleitersensoren nicht gemessen. Ebenso kann die Sensoreinheit auf Gasgemische mit hohen Inertgas-Anteilen, wie beispielsweise Stickstoff oder Kohlenstoffdioxid, angewendet werden.

Die Auswertung von Sensorsignalen des Ist-Gasbeschaffenheitsmusters kann ebenso über mathematische Methoden erfolgen und das dem aktuellen Ist-Gasbeschaffenheitsmuster nächstkommende Soll-Gasbeschaffenheitsmuster gefunden werden. Jeder einzelne Sensorwert kann mit einer Abweichung von höchstens ±1%, bevorzugt höchstens ±0,5% mit den bekannten Soll-Gasbeschaffenheitsmustern übereinstimmen, damit der Brennwert insgesamt mit einer Genauigkeit von ±2% bestimmt werden kann.

Da die Zusammensetzung des Gases, aus dem das Soll-Gasbeschaffenheitsmuster erzeugt wurde, bekannt ist, kann der aktuelle Brennwert bestimmt werden.

Niederkalorische Gase, so genannte L-Gase, können als Brenngase Wasserstoff (H₂) mit einem typischen Volumenanteil von kleiner 0,002 vol%, Methan (CH₄) mit 82 bis 88 vol%, Ethan (C₂H₆) mit 3 bis 7 vol%, Butan (C₄H₁₀) mit kleiner 0,5 vol%, Pentan (C₅H₁₂) mit kleiner 0,2 vol%, sowie als Inertgase Helium (He) mit kleiner 0,01 vol%, Kohlendioxid (CO₂) mit 0,5 bis 2,5 vol%, Stickstoff (N₂) mit 5 bis 15 vol% aufweisen.

Hochkalorische Gase, so genannte H-Gase, können als Brenngase Wasserstoff (H₂) mit einem typischen Volumenanteil von kleiner 0,001 vol%, Methan (CH₄) mit 93 bis 96 vol%, Ethan (C₂H₆) mit 1 bis 3 vol%, Butan (C₄H₁₀) mit kleiner 0,1 vol%, Pentan (C₅H₁₂) mit kleiner 0,02 vol%, sowie als Inertgase He mit kleiner 0,02 vol%, Kohlendioxid (CO₂) mit kleiner 0,3 vol%, Stickstoff (N₂) mit 1 bis 2 vol% aufweisen.

Wenn jedem Gasbestandteil eine Dimension x eines sogenannten "Gasbeschaffenheitsraumes" zugeordnet wird, entsteht ein x-dimensionaler Raum mit aus den Sensorsignalen bestimmten Soll-Gasbeschaffenheitsmustern, denen der jeweils bekannte Brennwert eines Referenzgases mit einem bestimmten Gasbestandteil x zugeordnet werden kann.

Den Soll-Gasbeschaffenheitsmustern können auch andere Eigenschaften des Gasgemisches des Referenzgases zugeordnet werden, bspw. der stöchiometrische Kohlenstoffgehalt, aus dem sich bei einer Verbrennung die CO₂-Emissionen ergeben. Den Soll-Gasbeschaffenheitsmustern können auch die genauen Anteile der Gasbestandteile zugeordnet werden, aus denen sich dann technisch-physikalische Eigenschaften später berechnen lassen.

Für den Zweck der Energieabrechnung ist es vollkommen ausreichend, von Den Soll-Gasbeschaffenheitsmustern direkt auf den Brennwert zu schließen. Für den Zweck einer Verbrennungssteuerung ist hingegen die genaue Kenntnis der Gaszusammensetzung zweckmäßig.

Nach einer günstigen Ausgestaltung kann eine Schrittweite einer Konzentrationsänderung eines dem Referenzgas beigemischten Gasbestandteils in der Lernphase beim Generieren der Soll-Gasbeschaffenheitsmuster so gewählt werden, dass Brennwert-Differenzen bei der Lernphase für alle Gasbestandteile kleiner sind als eine maximal zulässige Messtoleranz.

Die Schrittweite beim Tainieren der Sensoreinheit kann zweckmäßigerweise so gewählt werden, dass die Brennwert-Differenzen in allen Dimensionen kleiner sind als die maximal zulässige Messtoleranz. Die gemessenen Sensorsignale der Sensoreinheit stimmen üblicherweise nicht in allen Dimensionen völlig mit einem Sensorsignal des Referenzgases überein. Mittels mathematischer Regressionsverfahren kann aus den diskreten Soll-Gasbeschaffenheitsmustern ein "best fit" Soll-Gasbeschaffenheitsmuster ermittelt werden. Gegebenenfalls kann zwischen verschiedenen gleich "guten" Soll-Gasbeschaffenheitsmustern interpoliert werden. Die Interpolation kann nach gängigen mathematischen Verfahren erfolgen und kann zur Verbesserung der Genauigkeit auf Funktionen höherer Ordnung beruhen (bspw. Spline-Funktionen).

Nach einer günstigen Ausgestaltung kann dem Soll-Gasbeschaffenheitsmuster ein bei der Beaufschlagung mit dem Referenzgas herrschender Druck und/oder eine Temperatur zugeordnet werden. Als zusätzliche Dimensionen bzw. Parameter können zweckmäßigerweise auch Druck und Temperatur berücksichtigt werden. Im Zähler können dazu einfache Druck- und Temperaturgeber integriert werden.

Vorteilhaft ist auch, dass der Brennwert aus den Gasbestandteilen nicht analytisch "berechnet" werden muss, sondern dass ein bekannter Brennwert des als "Trainingsgas" verwendeten Referenzgases der Signalantwort der Sensoreinheit zugeordnet wird. Der Brennwert wird damit durch Vergleich mit dem Sensorsignal ermittelt, muss jedoch nicht errechnet werden, sondern allenfalls zwischen diskreten Messpunkten interpoliert werden.

Ein zentrales Element der üblichen Abrechnungsmethode für eine Gasabrechnung nach der Technischen Regel DVGW G685:2008 ist, dass die Mengenmessung, d.h. Volumenmessung, lokal und die Gasbeschaffenheitsmessung und Brennwertberechnung über einen Prozessgaschromatographen (PGC) zentral erfolgt. Abweichend davon erfolgt bei dem erfindungsgemäßen Verfahren nur die Lernphase für den Sensor zentral.

Die Sensoreinheit, bzw. der damit ausgerüstete Gaszähler liefert dann direkt den Brennwert, nämlich aus dem Vergleich des Signals der Sensoreinheit mit den hinterlegten Test-Signaturen.

Dadurch dass der Brennwert im Zähler lokal durch Vergleich der Ist-Gasbeschaffenheitsmustern mit den Soll-Gasbeschaffenheitsmustern der Sensoreinheit vorliegt und mit dem gemessenen Gasbezugsvolumenstrom verrechnet werden kann, kann auch der Energieverbrauch instantan am Zähler oder einem separaten Display angezeigt werden. Bisher können auch "elektronische" Gaszähler nur die Gasmenge auf einem elektronischen Display anzeigen, nicht aber die Energiemenge. Dazu ist eine Verrechnung mit dem lokal zu messenden Brennwert notwendig.

Nach einer günstigen Ausgestaltung kann zumindest der aktuelle Brennwert in einem festen Zeitintervall und/oder ereignisbezogen, insbesondere bei einer Veränderung des Brennwerts um mindestens 0,5%, vorzugsweise um mindestens 1%, an eine Zentraleinheit weitergeleitet werden. Dies kann bedarfsweise festgelegt werden.

Nach einer günstigen Ausgestaltung kann das Verfahren in sauerstoffarmer oder sauerstofffreier Umgebung eingesetzt werden. Übliche Halbleitersensoren werden in Sauerstoffatmosphäre eingesetzt, um Spurengase im ppb- oder ppm-Bereich in Luft nachzuweisen.

Erfindungsgemäß werden die Teilsensoren der Sensoreinheit mit geeigneten Aktivierungsmitteln wie Bestrahlung mit UV oder IR oder mittels einer Mikroheizung aktiviert.

Die Teilsensoren werden hierdurch für den Nachweis bestimmter Komponenten im Gas empfindlich gemacht.

Nach einer günstigen Ausgestaltung kann die Konzentration von wenigstens einem Gasbestandteil im Promillebereich oder Prozentbereich erfasst werden. Damit lassen sich typische Gaszusammensetzungen verschiedener Erdgasqualitäten erfassen und analysieren.

Nach einer günstigen Ausgestaltung kann die Sensoreinheit die Brennwerte für mehrere Zählereinrichtungen erfassen. Alternativ kann jeweils eine Sensoreinheit den Brennwert jeweils einer Zählereinrichtung erfassen. Damit können unterschiedliche Anforderungen an die Brennwerterfassung in unterschiedlichen Umgebungen einfach erfüllt werden. Beispielsweise kann ein Mehrfamilienhaus mit Gas versorgt sein, dessen Brennwert mit nur einer Sensoreinheit bestimmt wird und an dem der Verbrauch mit mehreren Zählereinrichtungen erfasst wird.

Nach einer günstigen Ausgestaltung können die Teilsensoren zum Generieren des jeweiligen Sensorsignals thermisch und/oder durch ein Aktivierungsgas und/oder durch Bestrahlung aktiviert werden. Dies kann für alle Teilsensoren in gleicher Weise erfolgen. Optional kann eine lokale Aktivierung von einzelnen Teilsensoren oder von Bereichen von Teilsensoren bedarfsgerecht erfolgen. Als Aktivierungsgas kann ein reduzierendes oder oxidierendes Medium eingesetzt werden. Das Aktivierungsgas kann lokal auf die Sensoreinheit einwirken und muss nicht im Erdgas enthalten sein.

Nach einer günstigen Ausgestaltung können die Teilsensoren das Ist-Gasbeschaffenheitsmuster kontinuierlich oder zumindest quasikontinuierlich erfassen. Dies erlaubt eine bedarfsgerechte Anpassung der Mess- oder Auswertefrequenz.

Nach einem weiteren Aspekt der Erfindung wird ein Verfahren vorgeschlagen zur Bestimmung einer Energiemenge eines Gasverbrauchs aus einem erfassten Volumenwert des Gasverbrauchs und einem Brennwert, wobei der Brennwert mit dem erfindungsgemäßen Verfahren bestimmt wird.

Auf teure Prozessgaschromatographie kann verzichtet werden. Ebenso können volatile Gaszusammensetzungen zuverlässig erfasst werden.

Nach einer günstigen Ausgestaltung kann zumindest der Brennwert kontinuierlich oder zumindest quasikontinuierlich erfasst werden. Dies erlaubt eine bedarfsgerechte Anpassung der Mess- oder Auswertefrequenz.

Nach einer günstigen Ausgestaltung kann zumindest der aktuelle Brennwert in einem festen Zeitintervall und/oder ereignisbezogen, insbesondere bei einer Veränderung des Brennwerts um mindestens 0,5%, vorzugsweise um mindestens 1%, an eine Zentraleinheit weitergeleitet werden. Dies erlaubt eine bedarfsgerechte Anpassung der Mess- oder Auswertefrequenz insbesondere bei volatilen Gaszusammensetzungen.

Nach einem weiteren Aspekt der Erfindung wird die Verwendung einer Sensoreinheit zur Durchführung eines Verfahrens zur Bestimmung des Brennwerts mit einer Sensoreinheit vorgeschlagen, wobei Teilsensoren der Sensoreinheit als Halbleitersensoren ausgebildet sind.

Nach einem weiteren Aspekt der Erfindung wird die Verwendung einer Sensoreinheit zur Durchführung eines Verfahrens zur Bestimmung einer Energiemenge eines Gasverbrauchs mit einer Sensoreinheit vorgeschlagen, wobei Teilsensoren der Sensoreinheit als Halbleitersensoren ausgebildet sind.

In beiden Fällen kann vorteilhaft eine volatile Gaszusammensetzung bestimmt werden. Die Messung der Gasbeschaffenheit und die Berechnung des Brennwerts, insbesondere eines Abrechnungsbrennwertes, sowie der zugehörigen Energiemenge gelingt somit mit einem einfachen und günstigen Messverfahren, welches weitestgehend miniaturisiert ist und vollständig in einen lokalen Gaszähler integriert sein kann oder bei Bedarf separat vorgesehen sein kann.

Bisher wird die Brennwertbestimmung mittels Prozessgaschromatographen durchgeführt. Diese nutzen Wasserstoff als Trägergas. Der Wasserstoffanteil im Gas kann deshalb mit den üblichen Prozessgaschromatographen nicht ermittelt werden. In der Zukunft ist daher ein hoher Anpassungsaufwand zu erwarten. Durch die beschriebene Erfindung kann dies langfristig entfallen.

Bei bisherigen Anwendungen einer solchen Sensoreinheit an Luft sorgt der Luftsauerstoff für oxidierende Bedingungen. Bei der Brennwertermittlung erfolgt die Messung in einer quasi sauerstofffreien Atmosphäre, die überwiegend aus Methan und höheren Kohlenwasserstoffen besteht.

Die Sensoreinheit besteht aus einer Anzahl unterschiedlich zusammengesetzter und/oder dotierter Teilsensoren, die durch UV-Bestrahlung und/oder IR-Bestrahlung und/oder eine Mikroheizung unterschiedlich aktiviert werden und durch die Anwesenheit reduzierender oder oxidierender Gasbestandteile eine messbare Widerstandsänderung erfahren.

Als Halbleiter werden Metalloxide bevorzugt eingesetzt, insbesondere SnO₂ und/oder ZnO. Es können auch andere Halbleiter, wie Al₂O₃, FeO, Fe₂O₃, WO₃, TiO₂, In₂O₃, Ga₂O₃ und dergleichen, eingesetzt werden, oder auch Kombinationen davon. Die Teilsensoren reagieren nicht selektiv nur auf ein einzelnes Gas, sondern weisen für verschiedene Gase unterschiedliche Sensitivitäten auf. Die Selektivität kann durch eine Modifikation der Teilsensoren hinsichtlich Material, Oberflächenstruktur, Körnung, Dicke, Additive, Edelmetall-Dotierung, Deckschicht und dergleichen, des Grundsubstrates, insbesondere des Materials und der Struktur der Elektroden, welche die Sensorsignale der Teilsensoren abgreifen sowie der Betriebsbedingungen, wie beispielsweise Druck, Temperatur, Elektrodenpotenzial, in weiten Bereichen beeinflusst werden.

Bei bekannten Halbleitersensoren auf Metalloxidbasis führen Sauerstoff-Fehlstellen in der Kristallstruktur der Halbleitermaterialien zu n-leitenden Eigenschaften, überschüssige Sauerstoffatome im Kristallgitter führen zu p-Typ-Halbleitern. Die Leitfähigkeit des Sensors ist damit abhängig von der Sauerstoff-Konzentration der Umgebung, bzw. allgemeiner von der Anwesenheit reduzierender oder oxidierender Gase im zu charakterisierenden Gas. An der Oberfläche des Halbleiters ist die Kristallstruktur prinzipiell gestört. Je nach Energieniveau der Störung und der Bandlücke des Halbleitermaterials können diese Störungen als Donatoren oder Akzeptoren Ladungen abgeben oder aufnehmen. Die Störung führt zur Ausbildung elektrisch unterschiedlich gepolter Schichten an der Oberfläche und im Innern des Halbleiterkristalls in Form von Raumladungszonen und letztlich den Ladungsaustausch mit nicht-inerten Gasatomen, die teilweise auch in das Kristallgitter eindiffundieren und dort zur Änderung des elektrischen Widerstands führen.

Durch den Einsatz vieler unterschiedlich sensibler Einzelsensoren kann die Trennschärfe der Mustererkennung der Gasbeschaffenheit so gesteigert werden, dass auch Gasgemische mit zehn oder mehr Komponenten im Prozentbereich oder Promille-Bereich, wie sie typisch für Erdgas sind, mit einer ausreichenden Genauigkeit bestimmt werden können.

Nach einem weiteren Aspekt der Erfindung wird eine Vorrichtung zur Durchführung des Verfahrens zur Bestimmung eines Brennwerts eines methanhaltigen Brenngases vorgeschlagen, wobei wenigstens eine Sensoreinheit mit einer Mehrzahl von Teilsensoren vorgesehen ist, wobei unterschiedliche Teilsensoren eingerichtet sind unterschiedlich sensitiv auf denselben Gasbestandteil zu reagieren und/oder derselbe Teilsensor eingerichtet ist auf unterschiedliche Konzentrationen eines jeweils gleichen Gasbestandteils in der Gaszusammensetzung zu reagieren.

Die Sensoreinheit besteht aus einer Anzahl unterschiedlich zusammengesetzter und/oder dotierter Teilsensoren. Die Teilsensoren werden durch eine Strahlungsquelle durch UV-Bestrahlung und/oder IR-Bestrahlung oder durch eine Mikroheizung unterschiedlich aktiviert. Die Teilsensoren erfahren durch die Anwesenheit reduzierender oder oxidierender Gasbestandteile eine messbare Widerstandsänderung.

Eine Signalerfassungsinstanz ist vorgesehen, die eingerichtet ist die Widerstandsänderungen als Sensorsignale der jeweiligen Teilsensoren zu erfassen, die von der Gaszusammensetzung und/oder der Konzentration von einem oder mehreren Gasbestandteilen abhängig ist, und wobei Teilsensoren und Sensorsignal in einem Ist-Gasbeschaffenheitsmuster repräsentiert sind.

Weiter ist eine Auswerteinstanz vorgesehen, welche eingerichtet ist ein nächstkommendes Soll-Gasbeschaffenheitsmuster des Soll-Gasbeschaffenheitsmusters zu bestimmen und daraus einen aktuellen Brennwert zu extrahieren. Hierbei erfolgt die Bestimmung des aktuellen Brennwerts des Brenngases aus dem nächstkommenden Soll-Gasbeschaffenheitsmuster statt durch analytische Berechnung durch Vergleich des durch die Sensoreinheit gelieferten Ist-Gasbeschaffenheitsmusters mit dem nächstkommenden Soll-Gasbeschaffenheitsmuster oder mittels Interpolation aus mehreren Soll-Gasbeschaffenheitsmustern.

Ferner ist eine Kommunikationsinstanz vorgesehen, die eingerichtet ist den extrahierten aktuellen Brennwert an eine Zentraleinheit zu übermitteln.

Die Teilsensoren der Sensoreinheit sind als Halbleitersensoren ausgebildet. Durch den Einsatz vieler unterschiedlich sensibler Einzelsensoren kann die Trennschärfe der Mustererkennung der Gasbeschaffenheit so gesteigert werden, dass auch Gasgemische mit zehn oder mehr Komponenten im Prozentbereich oder Promille-Bereich, wie sie typisch für Erdgas sind, mit einer ausreichenden Genauigkeit bestimmt werden können.

Die Zentraleinheit kann eine lokale Einheit oder eine entfernte Einheit sein.

Nach einem weiteren Aspekt der Erfindung wird eine Vorrichtung zur Durchführung des Verfahrens zur Bestimmung einer Energiemenge eines Gasverbrauchs vorgeschlagen, wobei wenigstens eine Sensoreinheit mit einer Mehrzahl von Teilsensoren vorgesehen ist, wobei unterschiedliche Teilsensoren unterschiedlich sensitiv auf denselben Gasbestandteil reagieren und/oder derselbe Teilsensor auf unterschiedliche Konzentrationen eines jeweils gleichen Gasbestandteils in der Gaszusammensetzung reagiert. Die Sensoreinheit besteht aus einer Anzahl unterschiedlich zusammengesetzter und/oder dotierter Teilsensoren, die durch UV-Bestrahlung und/oder IR-Bestrahlung und/oder eine Mikroheizung unterschiedlich aktiviert werden und durch die Anwesenheit reduzierender oder oxidierender Gasbestandteile eine messbare Widerstandsänderung erfahren. Eine Signalerfassungsinstanz ist vorgesehen, die eingerichtet ist die Widerstandsänderungen als jeweiliges Sensorsignal zu erfassen, das von der Gaszusammensetzung und/oder der Konzentration von einem oder mehreren Gasbestandteilen abhängig ist, wobei das jeweilige Sensorsignal dem jeweiligen Teilsensor in einem Ist-Gasbeschaffenheitsmuster zugeordnet ist. Weiter ist eine Auswerteinstanz vorgesehen, welche ein nächstkommendes Soll-Gasbeschaffenheitsmuster des Ist-Gasbeschaffenheitsmusters bestimmt und daraus einen aktuellen Brennwert extrahiert. Hierbei erfolgt die Bestimmung des aktuellen Brennwerts des Brenngases aus dem nächstkommenden Soll-Gasbeschaffenheitsmuster statt durch analytische Berechnung durch Vergleich des durch die Sensoreinheit gelieferten Ist-Gasbeschaffenheitsmusters mit dem nächstkommenden Soll-Gasbeschaffenheitsmuster oder mittels Interpolation aus mehreren Soll-Gasbeschaffenheitsmustern.

Ferner ist ein Zählwerk zur Erfassung eines Gasvolumens vorgesehen. Weiterhin ist eine Kommunikationsinstanz vorgesehen, die die bestimmte Energiemenge an eine Zentraleinheit übermittelt.

Die Zentraleinheit kann eine lokale Einheit sein, in der eine lokale Verrechnung mit der Volumenmessung erfolgt oder eine entfernte Einheit, in der die Verrechnung eines Brennwerts mit der Volumenmessung von verschiedenen Gasmengenzählern erfolgt.

Die Kommunikationsinstanz und/oder die Auswerteinstanz können als Hardware-Einheit sein oder auch ganz oder teilweise in Software realisiert sein.

Zur Bestimmung der Energiemenge wird zweckmäßigerweise das verbrauchte Gasvolumen mit einem Zählwerk eines Gaszählers erfasst und mit dem Brennwert zu der aktuellen Energiemenge verknüpft.

Bei Gaszählern kommen über den weiten Bereich der möglichen Volumenströme die verschiedensten Messverfahren zur Anwendung. Zu den Verdrängungsgaszählern zählen neben Balgengaszählern auch Trommel-, Drehkolben- oder Drehschleusenzähler. Zu den Strömungsgaszählern zählen Turbinenrad-, Drall-, Ultraschall- und Wirbelgaszähler. Sonderbauformen sind Wirkdruck-, Coriolis- und elektronische Gaszähler.

Vorteilhaft kann eine vollständig dezentrale Gasabrechnung durch voll integrierte elektronische Gaszähler erfolgen.

Sowohl Brennwert als auch die Energiemenge eines Gasverbrauchs kann für volatile Gaszusammensetzungen mit hoher Präzision bestimmt werden.

Besonders vorteilhaft gelingt eine einfache und kostengünstige Lösung mit Halbleiter-Gassensoren, die in der Gasmesstechnik bereits lange erprobt sind. Allerdings dienen die konventionellen Halbleiter-Gassensoren dort dazu, Spurengase beispielsweise in der Atemluft nachzuweisen.

Nach einer günstigen Ausgestaltung der jeweiligen Vorrichtung kann die Auswerteinstanz und/oder die Kommunikationsinstanz mit einem Zählwerk für ein Verbrauchsvolumen an Brenngas gekoppelt sein.

Dies erlaubt die Erfassung von verbrauchten Gasvolumina und eine entsprechende genaue Abrechnung der Energiemenge auch bei volatilen Gaszusammensetzungen.

Nach einer günstigen Ausgestaltung der jeweiligen Vorrichtung können zumindest die Auswerteinstanz, die Kommunikationsinstanz und das Zählwerk in einer gemeinsamen Zählereinrichtung angeordnet sein. Dies ergibt eine kompakte. eigenständige Einheit, die einfach auch in bestehende Systeme integriert werden kann. Insbesondere kann die Zählereinrichtung eine Einheit aufweisen, welche die Auswerteinstanz, die Kommunikationsinstanz sowie eine Recheneinheit umfasst. Hiermit kann eine Brennwertberechnung und/oder Energiemenge des Gasverbrauchs berechnet werden.

Nach einer günstigen Ausgestaltung der jeweiligen Vorrichtung kann eine Sensoreinheit mehreren Zählereinrichtungen zugeordnet sein. Dies ist beispielsweise für ein Mehrfamilienhaus mit einer Wohnung je Etage günstig. Die Sensoreinheit kann nahe des Hausanschlusses angeordnet sein und die Bestimmung der Volumenströme je Wohneinheit auf den einzelnen Etagen mittels des jeweiligen Gaszählers.

Nach einer günstigen Ausgestaltung der jeweiligen Vorrichtung kann alternativ jeweils eine Sensoreinheit jeweils einer Zählereinrichtung zugeordnet sein. Es kann eine vollständig unabhängige Erfassung von Brennwert und/oder Verbrauch erfolgen.

Vorteilhaft können die erfindungsgemäße Vorrichtung und das erfindungsgemäße Verfahren auch bei sensiblen Anwendungen zum Einsatz kommen, so dass in Echtzeit auf unterschiedliche Gasbeschaffenheiten reagiert werden kann. Solche sensiblen Anwendungen sind beispielsweise bei sensiblen Thermoprozessanlagen in der Glas- oder Keramikindustrie zu finden.

Mit der Brennwertbestimmung in der Zählereinrichtung wird auch eine lokale Darstellung des Energieverbrauchs in Echtzeit im Sinne eines "smart gas-meters" möglich, bei der ein Verbraucher den Verbrauch und die Kosten in Echtzeit verfolgen kann.

Durch den Einsatz vieler unterschiedlich sensibler Einzelsensoren kann die Trennschärfe der Mustererkennung so gesteigert werden, dass auch Gasgemische mit zehn oder mehr Komponenten im Prozent- oder Promille-Bereich, wie sie typisch für Erdgas sind, mit einer ausreichenden Genauigkeit bestimmt werden können.

### Zeichnung

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen beispielhaft:

- Fig. 1: schematisch ein Funktionsprinzip einer Sensoreinheit nach einem Ausführungsbeispiel der Erfindung;
- Fig. 2: Messkurven mit Sensorsignalen eines Teilsensors nach einem Ausführungsbeispiel der Erfindung;
- Fig. 3: mehrere Gasbeschaffenheitsmuster mit Sensorsignalen von sieben Teilsensoren, die einem Referenzgas mit einem Gasbestandteil mit verschiedenen Konzentrationen ausgesetzt wurden;
- Fig. 4: mehrere Gasbeschaffenheitsmuster mit Sensorsignalen von sieben Teilsensoren in unterschiedlichen Referenzgasen;
- Fig. 5: eine Zählereinrichtung mit einer Sensoreinheit nach einem Ausführungsbeispiel der Erfindung;
- Fig. 6: schematisch ein Mehrfamilienhaus mit einer zentralen Sensoreinheit nach einem Ausführungsbeispiel der Erfindung und dezentralen Zählereinrichtungen;
- Fig. 7: schematisch ein Mehrfamilienhaus mit dezentralen Zählereinrichtungen mit jeweils einer integrierten Sensoreinheit nach einem Ausführungsbeispiel der Erfindung.

### Ausführungsformen der Erfindung

In den Figuren sind gleichartige oder gleichwirkende Komponenten mit gleichen Bezugszeichen beziffert. Die Figuren zeigen lediglich Beispiele und sind nicht beschränkend zu verstehen.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

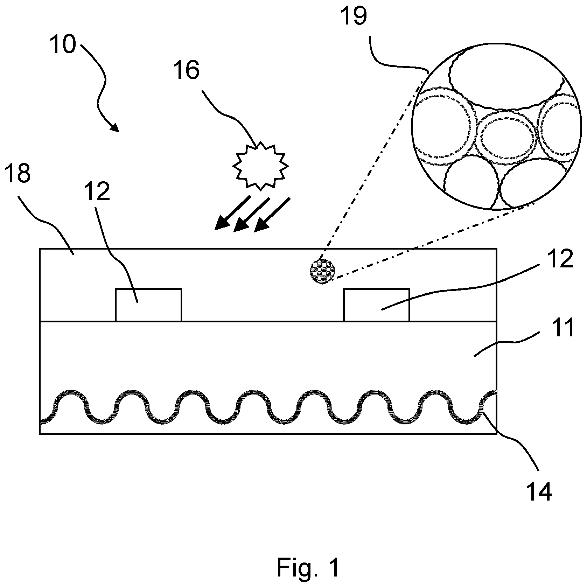

Figur 1 zeigt schematisch ein Funktionsprinzip einer Sensoreinheit 10 nach einem Ausführungsbeispiel der Erfindung dar. Eine Halbleiterschicht 18 ist auf einem Substrat 11 abgeschieden. Unter der Halbleiterschicht 18 sind Elektroden 12 angeordnet, mit denen Sensorsignale der Halbleiterschicht 18 abgegriffen werden können.

Es können weitere elektronische Schaltkreise auf der Sensoreinheit 10 angeordnet sein, etwa zur Signalerfassung und/oder Mikroprozessoren zur Auswertung der Sensorsignale und zur Berechnung des Brennwerts. Alternativ kann die Sensoreinheit 10 mit derartigen Komponenten verbunden sein, insbesondere in einem Gehäuse integriert sein.

Die Sensoreinheit 10 umfasst eine Anzahl in der Figur nicht näher ausgeführter unterschiedlich zusammengesetzter und/oder dotierter Teilsensoren. Diese können als durchgehende Beschichtung auf dem Substrat 11 gebildet sein, die lokal unterschiedlich dotiert ist oder auch als voneinander getrennte separate Halbleiterflächen.

Die Teilsensoren werden mittels einer Strahlungsquelle 16 durch UV-Licht, IR-Strahlung, oder mittels einer Mikroheizung 14 unterschiedlich aktiviert, was die Bandlücke des Halbleiters variiert, sodass die Teilsensoren durch die Anwesenheit reduzierender oder oxidierender Gasbestandteile eine messbare Widerstandsänderung erfahren, welche als Sensorsignale über die Elektroden 12 abgegriffen werden können.

Als Halbleiter können Metalloxide (bevorzugt SnO₂ und/oder ZnO, aber auch Al₂O₃, FeO, Fe₂O₃, WO₃, TiO₂, In₂O₃, Ga₂O₃, etc.) oder auch Kombinationen davon eingesetzt werden. Die Teilsensoren reagieren nicht selektiv nur auf ein einzelnes Gas, sondern weisen für verschiedene Gase unterschiedliche Sensitivitäten auf. Die Selektivität kann durch Modifikation der Schichten (Material, Oberflächenstruktur, Körnung, Dicke, Additive, Edelmetall-Dotierung, Deckschicht), des Grundsubstrates (Material und Struktur der Elektroden) sowie der Betriebsbedingungen (Druck, Temperatur, Elektrodenpotenzial) in weiten Bereichen beeinflusst werden.

Sauerstoff-Fehlstellen in der Kristallstruktur der Halbleitermaterialien führen zu n-leitenden Eigenschaften, überschüssige Sauerstoffatome im Kristallgitter führen zu p-Typ-Halbleitern. Die Leitfähigkeit des Sensors ist damit abhängig von der Sauerstoff-Konzentration der Umgebung. An der Oberfläche des Halbleiters ist die Kristallstruktur prinzipiell gestört. Je nach Energieniveau der Störung und der Bandlücke des Halbleitermaterials können diese Störungen als Donatoren oder Akzeptoren Ladungen abgeben oder aufnehmen. Die Störung führt zur Ausbildung elektrisch unterschiedlich gepolter Schichten an der Oberfläche und im Innern des Kristalls (Raumladungszonen) und letztlich den Ladungsaustausch mit nicht-inerten Gasatomen, die teilweise auch in das Kristallgitter eindiffundieren und dort zur Änderung des elektrischen Widerstands führen.

Zum Nachweis verschiedener Gase oder Dämpfe sind Oxide der nachfolgend in der Tabelle aufgeführten Metalle geeignet und für den jeweilig nachweisbaren Stoff durch ein X gekennzeichnet:

| Gas | Al | Ga | Fe | Sn | Zn |
|---|---|---|---|---|---|
| Benzol (C₆H₆) | | | | X | |
| Butan (C₄H₁₀) | X | X | X | X | X |
| Ethan (C₂H₆) | | | | | X |
| Methylmerkaptan (CH₃SH) | | | | X | |
| Kohlendioxid (CO₂) | X | | X | X | X |
| Schwefelwasserstoff (H₂S) | | | | X | X |
| Propan (C₃H₈) | | X | X | X | X |
| Wasser (H₂O) | X | | X | X | X |
| Wasserstoff (H₂) | X | | X | X | X |

Die Metalloxide können in üblicher Weise durch nasschemischen Auftrag und Tempern, nach dem Aerogel-Verfahren, physikalisch (z.B. Sputtern) oder Abscheiden aus der Dampfphase aufgebracht werden.

Die Anzahl und Form der Kontaktflächen der einzelnen Halbleiter-Kristalle bestimmt u.a. die Leitfähigkeit. Die Größe der Kontaktflächen ist abhängig von der Partikelgröße und reicht vom mikroskaligen bis zum nanoskaligen Bereich, so dass mit der Wahl des Herstellungsverfahrens die Sensitivität des Sensors auf das zu messende Gasgemisch abgestimmt werden kann. Dies ist in der Figur in einer Detailvergrößerung 19 angedeutet.

Der innere Aufbau, die Leitfähigkeit und die Ausbildung einer Ladungsverarmungszone (gestrichelt in der Detailvergrößerung 19) an den Kontaktstellen des Halbleiters hängen sehr stark vom Herstellungsverfahren ab.

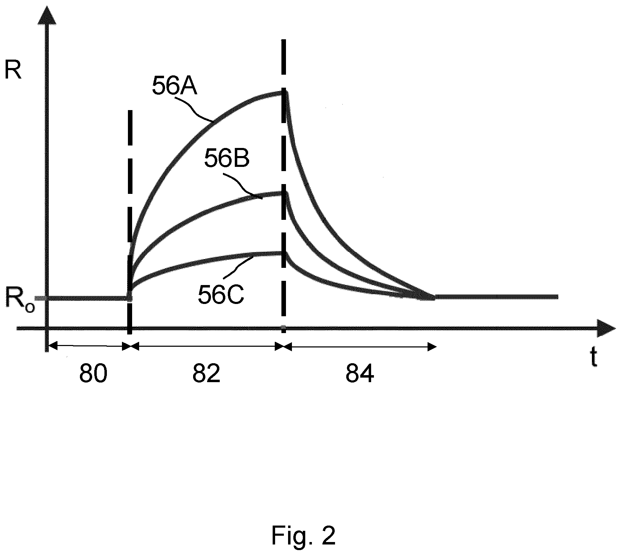

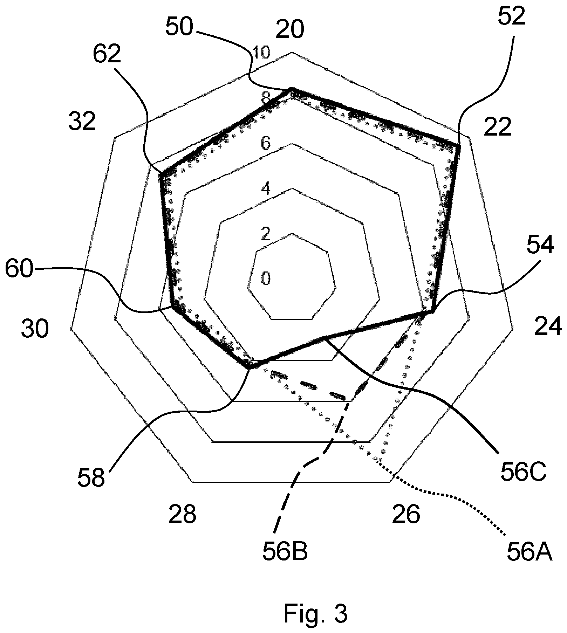

Wird die Sensoreinheit 10 mit einem oxidierenden oder reduzierenden Gas beaufschlagt, beeinflusst dies die Raumladungszonen und verändert so den Widerstand des Halbleiters. Die Sprungantwort des Sensors ist dabei von der Konzentration des zu detektierenden Gases abhängig, wie dies in Figur 2 und Figur 3 dargestellt ist.

Figur 2 zeigt Messkurven mit Sensorsignalen 56A, 56B, 56C eines Teilsensors 26 (Figur 3) nach einem Ausführungsbeispiel der Erfindung, und Figur 3 zeigt mehrere Gasbeschaffenheitsmuster mit Sensorsignalen von sieben Teilsensoren 20, 22, 24, 26, 28, 30, 32, die mehrmals einem Referenzgas bzw. Gasgemisch ausgesetzt wurden, das bei jeder Exposition zusätzlich mit einem bestimmten Gasbestandteil in unterschiedlicher Konzentration beaufschlagt wird.

Wie in Figur 2 zu erkennen ist, reagiert der Teilsensor 26 ausgehend von einer Basislinie im Bereich 80 in dem Referenzgas auf die Exposition mit dem bestimmten Gasbestandteil im Bereich 82, während der Teilsensor 26 im Bereich 84 in einer Erholungsphase nur noch dem Referenzgas und nicht mehr dem bestimmten Gasbestandteil ausgesetzt ist. Im Bereich 82 steigt das jeweilige Sensorsignal 56A, 56B, 56C abhängig von der Höhe der Konzentration des bestimmten Gasbestandteils im Referenzgas unterschiedlich stark an. Das Sensorsignal 56A zeigt eine hohe Konzentration von z.B. 1000 ppm des Gasbestandteils, Sensorsignal 56B eine mittlere Konzentration von z.B. 300 ppm und das Sensorsignal 56C eine geringe Konzentration von z.B. 100 ppm an.

Die Figur 3 zeigt eine Auftragung von Sensorsignalen 50, 52, 54, 56, 56A, 56B, 56C, 58, 60 in einem konzentrischen n-Eck, wobei n der Zahl der Teilsensoren 20, 22, 24, 26, 28, 30, 32 entspricht. Der Abstand vom Mittelpunkt entspricht der Signalgröße.

Während die Teilsensoren 20, 22, 24, 28, 30, 32 nicht auf verschiedene Konzentrationen des Gasbestandteils im Referenzgas reagieren und deren Sensorsignale 50, 52, 54, 58, 60, 62 konstant bleiben, zeigt der Teilsensor 26 die in Figur 2 beschriebene Abhängigkeit seiner Sensorsignale 56A, 56B, 56C.

Die Sensorsignale 50, 52, 54, 56A, 56B, 56C, 58, 60, 62 bilden ein Gasbeschaffenheitsmuster, das für die Konzentration des betreffenden Gasbestandteils im Referenzgas repräsentativ ist, auf den der Teilsensor 26 reagiert.

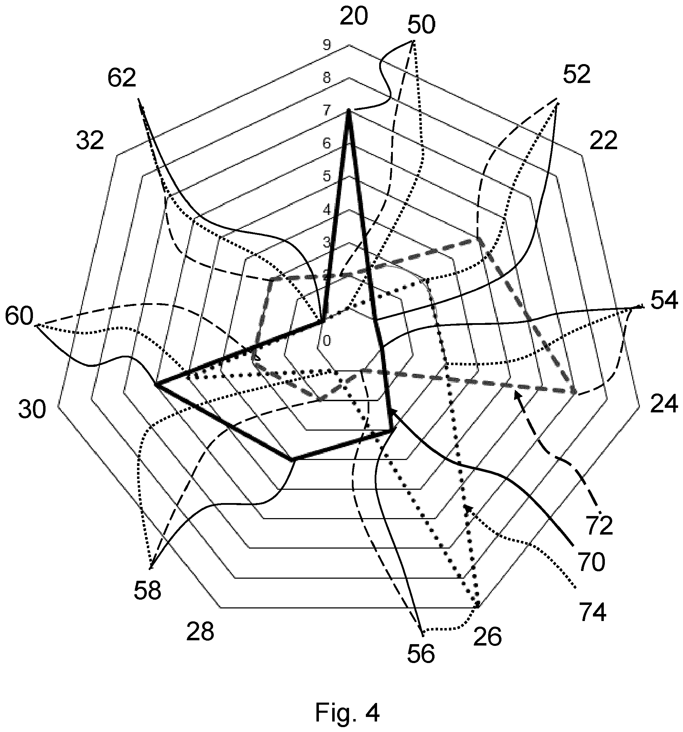

Figur 4 zeigt in ähnlicher Darstellung drei Gasbeschaffenheitsmuster für drei unterschiedliche Referenzgase 70, 72, 74, die jeweils unterschiedliche Gasbestandteile enthalten, mit Sensorsignalen 50, 52, 54, 56, 58, 60 von sieben Teilsensoren 20, 22, 24, 26, 28, 30, 32, die den drei Referenzgasen 70, 72, 74 ausgesetzt wurden.

Die einzelnen Teilsensoren 20, 22, 24, 26, 28, 30, 32 werden von den Gasbestandteilen eines Gasgemisches unterschiedlich stark angeregt. Ansonsten weitgehend identische Gasgemische können anhand der Konzentrationsunterschiede einer einzigen Komponente erkannt werden, wie dies schematisch für Teilsensor 26 in Figur 2 dargestellt ist. Der Teilsensor 26 spricht auf verschiedene Konzentrationen einer Gaskomponente unterschiedlich stark an.

Die Sensorsignale 50, 52, 54, 58, 60, 62 für das Referenzgas 70 sind mit durchgezogenen Linien im Diagramm eingezeichnet. Die Sensorsignale 50, 52, 54, 58, 60, 62 für das Referenzgas 72 sind mit strichlierten Linien im Diagramm eingezeichnet. Die Sensorsignale 50, 52, 54, 58, 60, 62 für das Referenzgas 74 sind mit punktierten Linien im Diagramm eingezeichnet.

Die drei Gasbeschaffenheitsmuster sind repräsentativ für das jeweilige Referenzgas 70, 72, 74, auf welche die Teilsensoren 20, 22, 24, 26, 28, 30, 32 mehr oder weniger stark reagieren.

Bei komplexen Gasgemischen entsteht ein charakteristisches "Muster". Eine Sensoreinheit mit beispielsweise sieben Teilsensoren 20, 22, 24, 26, 28, 30, 32 kann anhand dieser Anregung drei charakteristische Gase 70, 72, 74 gut unterscheiden, wie dies in Figur 4 schematisch dargestellt ist.

Die entstehenden "Gasbeschaffenheitsmuster" in der Widerstands- und/oder Spannungsänderung der Teilsensoren 20, 22, 24, 26, 28, 30, 32 können durch eine mathematische Auswertung beispielsweise mittels neuronaler Netze erkannt und einzelnen, auch komplexen Gasgemischen eindeutig zugeordnet werden. Auf diese Weise wird das System "trainiert". Im konkreten Anwendungsfall können auch Bestandteile wie bspw. Benzol, Toluol oder Xylole in die Analyse einbezogen werden, die in der bisherigen Brennwert-Bestimmung aufgrund ihres geringen Anteils keine Rolle spielen.

Die genaue Zusammensetzung und damit auch der Brennwert jedes Gasgemisches in der Lernphase ist bekannt und kann im Rechenwerk einer Zählereinrichtung hinterlegt werden. Für die weitere Verwertung des derart bestimmten Brennwerts zur Bestimmung der abzurechnenden Energiemenge gibt es verschiedene Möglichkeiten. Neben dem Brennwert wird hierfür das Volumen des Gases bestimmt. Dieses kann mittels der generell bekannten Verfahren bestimmt werden.

Zur Bestimmung eines Brennwerts eines Brenngases, das ein oder mehrere Gasbestandteile in seiner Gaszusammensetzung aufweist, wird demnach eine Sensoreinheit 10, die eine Mehrzahl von Teilsensoren 20, 22, 24, 26, 28, 30, 32 umfasst, mit dem Brenngas beaufschlagt, wobei unterschiedliche Teilsensoren 20, 22, 24, 26, 28, 30, 32 unterschiedlich sensitiv auf denselben Gasbestandteil reagieren und/oder derselbe Teilsensor 20, 22, 24, 26, 28, 30, 32 auf unterschiedliche Konzentrationen eines gleichen Gasbestandteils in der Gaszusammensetzung reagiert. Die von den Teilsensoren 20, 22, 24, 26, 28, 30, 32 erzeugten jeweiligen Sensorsignale 50, 52, 54, 56, 58, 60, 62, die von der Gaszusammensetzung und/oder der Konzentration von einem oder mehreren Gasbestandteilen abhängig sind, werden erfasst.

Das jeweilige Sensorsignal wird mit dem jeweiligen Teilsensor in einem Ist-Gasbeschaffenheitsmuster repräsentiert. Verschiedene Gaszusammensetzungen ergeben verschiedene Ist-Gasbeschaffenheitsmuster.

Es wird ein nächstkommendes Soll-Gasbeschaffenheitsmuster aus einer Gruppe von Soll-Gasbeschaffenheitsmustern bestimmt, beispielsweise mittels einer Analyse durch neuronale Netze. Die konkrete Gaszusammensetzung und/oder Konzentration von einem oder mehreren Gasbestandteilen wird aus dem nächstkommenden Soll-Gasbeschaffenheitsmuster extrahiert und daraus der aktuelle Brennwerts des Brenngases aus dem Soll-Gasbeschaffenheitsmuster durch Vergleich mit dem nächstkommenden Soll-Gasbeschaffenheitsmuster oder mittels Interpolation aus mehreren Soll-Gasbeschaffenheitsmustern bestimmt.

Die Soll-Gasbeschaffenheitsmuster können in einer Lernphase mittels mathematischer Methoden, insbesondere mittels neuronaler Netze, generiert werden, indem die Sensoreinheit mit den Teilsensoren verschiedensten Referenzgasen ausgesetzt wird und die Sensorsignale entsprechend erfasst und zu Gasbeschaffenheitsmustern zusammengefügt werden.

Die Soll-Gasbeschaffenheitsmuster können generiert werden, indem die Sensoreinheit mit Referenzgasen mit einer vorgegebenen Konzentration an ein oder mehreren Gasbestandteilen beaufschlagt wird und den erhaltenen Sensorsignalen als Soll-Gasbeschaffenheitsmuster ein jeweils bekannter Brennwert des Referenzgases zugeordnet wird.

Eine Schrittweite einer Konzentrationsänderung eines dem Referenzgas beigemischten Gasbestandteils kann zweckmäßigerweise in der Lernphase beim Generieren der Soll-Gasbeschaffenheitsmuster so gewählt werden, dass Brennwert-Differenzen bei der Lernphase für alle Gasbestandteile kleiner sind als eine maximal zulässige Messtoleranz.

Zusätzlich kann dem Soll-Gasbeschaffenheitsmuster ein bei der Beaufschlagung mit dem Referenzgas herrschender Druck und/oder eine Temperatur zugeordnet werden.

Die Energiemenge, die abgerechnet werden kann, lässt sich bestimmen, indem noch die Volumen-Messwerte in einer geeigneten Zählereinrichtung bestimmt werden und mit dem Brennwert multipliziert oder in sonstiger bekannter Weise erzeugt werden.

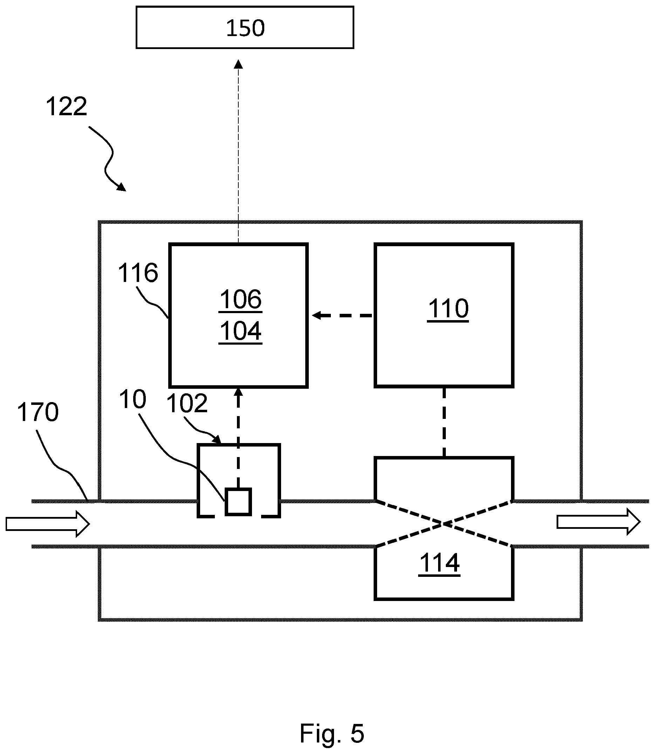

Figur 5 zeigt eine Zählereinrichtung 122 mit einer Sensoreinheit 10 nach einem Ausführungsbeispiel der Erfindung.

Für die Übermittlung des Zählerstands ist dabei eine elektronische Komponente 116 sowie eine Kommunikationsinstanz 106, z.B. ein Kommunikationsmodul, vorgesehen. Weiter ist eine Auswerteinstanz 104 vorgesehen, welche ein nächstkommendes Soll-Gasbeschaffenheitsmuster des Soll-Gasbeschaffenheitsmusters bestimmt und daraus einen aktuellen Brennwert extrahiert.

Das Zählwerk 110 in der Zählereinrichtung 122 zur Volumenbestimmung kann entweder direkt in einer Einheit mit der Sensoreinheit 10 mit Teilsensoren installiert sein, oder separat davon.

Die Messungen und deren Verrechnung kann entweder in einem festen Zeitintervall, beispielsweise minütlich oder viertelstündig, erfolgen oder ereignisbezogen erfolgen, also zum Beispiel bei jeder deutlichen Veränderung des ständig ermittelten Brennwerts.

Bei einer separaten Messwertbestimmung kann der Zählerstand des Zählwerks 110 an eine Recheneinheit 116 der Sensoreinheit 10 übermittelt und dort direkt mit dem Brennwert zu einer Energiemenge verrechnet werden. Dabei sind im Gasstrom jeweils eine Erfassung des Gasvolumenstromes in einem Messvolumen 114 und eine Erfassung des Gasbrennwerts über eine Signalerfassungsinstanz 102 der Sensoreinheit 10 integriert. Beide geben ihre Messwerte an eine in der Zählereinrichtung 122 integrierte Rechnereinheit 116 weiter, die den Energieverbrauch schließlich an eine Zentraleinheit 150, beispielsweise einen Gasversorger, weitersendet. Die Messung des Gasvolumenstroms im Messvolumen 114 kann dabei prinzipiell durch alle bekannten Zählerarten erfolgen.

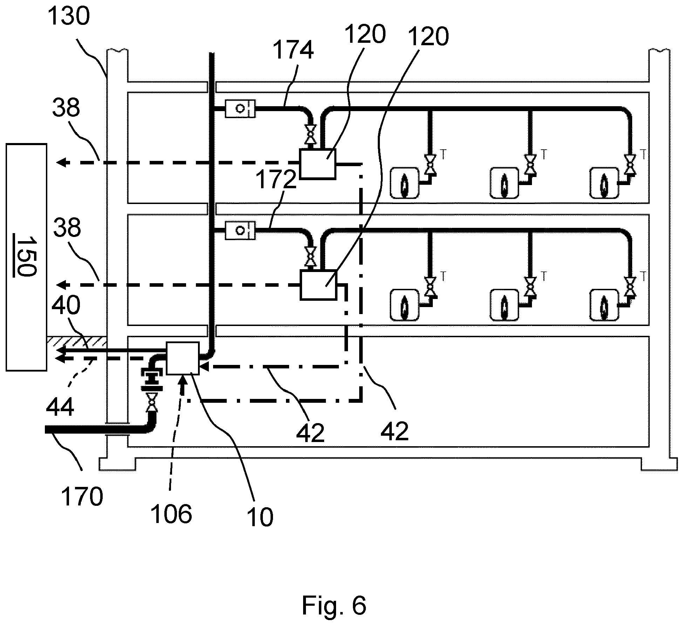

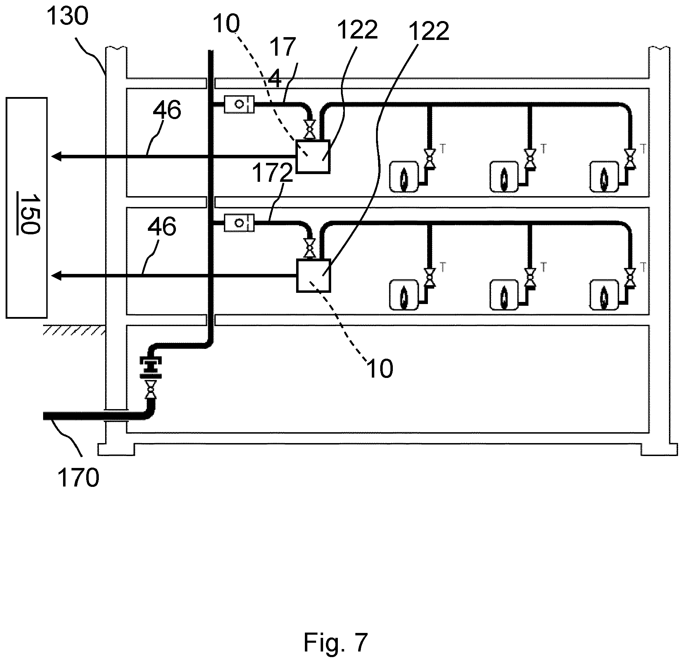

Die Figuren 6 und 7 illustrieren zwei Beispiele, wie ein aktueller Brennwert und/oder eine Energiemenge an Gasverbrauch an eine Zentraleinheit 150, beispielsweise einen Gasversorger oder eine zentrale Abrechnungsstelle, erfasst und übermittelt werden kann. Die Figuren zeigen schematisch ein Mehrfamilienhaus 130 mit mehreren Etagen, von denen zwei beispielhaft dargestellt sind. Jede Etage wird über einen Teilstrang 172, 174 mit Erdgas versorgt, das über eine Gasversorgungsleitung 170 in das Mehrfamilienhaus 130 geliefert wird. An die Teilstränge 172, 174 sind ein oder mehrere, nicht näher bezeichnete Gasbrenner angeschlossen.

Figur 6 zeigt eine zentrale Sensoreinheit 10 nach einem Ausführungsbeispiel der Erfindung mit dezentralen Zählereinrichtungen 120 in den verschiedenen Etagen zur Erfassung des Gasvolumens, das in der Etage verbraucht wird. Figur 7 zeigt dezentrale Zählereinrichtungen 122 mit jeweils einer integrierten Sensoreinheit 10 nach einem Ausführungsbeispiel der Erfindung. Der Gasverbrauch wird als Volumen in Zählereinrichtungen 120 (Figur 6) oder 122 (Figur 7) mittels Zählwerken 110 (Figur 5) erfasst.

In Figur 6 können die in der Zählereinrichtung 120 erfassten Gasvolumina separat über Signalpfade 38 drahtlos oder drahtgebunden an die Zentraleinheit 150 übermittelt werden. Die Sensoreinheit 10 mit den Halbleiterteilsensoren ist nahe zum Hausversorgungsanschluss in der Gasversorgungsleitung 170 beispielsweise im Keller des Mehrfamilienhauses 130 angeordnet und umfasst zur Weitergabe von Signalen mit einer nicht explizit dargestellten Kommunikationsinstanz 106 gekoppelt. Dort wird in der bereits beschriebenen Weise ein aktueller Brennwert des angelieferten Gases ermittelt und kann separat über einen Signalpfad 40 an die Zentraleinheit 105 weitergeleitet werden. Zweckmäßigerweise sind sowohl die erfassten verbrauchten Gasvolumina als auch die aktuellen Brennwerte mit entsprechenden Zeitstempeln versehen. In der Zentraleinheit kann aus Brennwerten und Volumina die Energiemenge berechnet werden, die den Verbrauchern in Rechnung gestellt werden können.

Alternativ kann die Verknüpfung von Brennwert und Volumen in der Sensoreinheit 10 als zentraler Einheit erfolgen. Hierzu wird der Sensoreinheit 10 der Gasvolumenstrom jeder Etage über Signalpfade 42 drahtgebunden oder drahtlos übermittelt, dort mit dem Brennwert verknüpft und über einen Signalpfad 44 an die Zentraleinheit 150 zur Verrechnung weitergeleitet. Die Sensoreinheit 10 weist in diesem Fall eine Einheit 116 wie in Figur 5 beschrieben auf. Insbesondere sind in diesem Fall entsprechende Soll-Gasbeschaffenheitsmuster in der Einheit 116 abgelegt.

In Figur 7 erfolgt die Verknüpfung von Brennwert und Gasvolumen zur Berechnung der Energiemenge direkt in jeder Zählereinheit 122. Die Zählereinheit 122 ist entsprechend dem Ausführungsbeispiel in Figur 5 ausgebildet.

### Bezugszeichen

- 10: Sensoreinheit
- 11: Substrat
- 12: Elektrode
- 14: Heizelement
- 16: Strahlungsquelle
- 18: Halbleiterschicht
- 19: Detailvergrößerung
- 20: Teilsensor
- 22: Teilsensor
- 24: Teilsensor
- 26: Teilsensor
- 28: Teilsensor
- 30: Teilsensor
- 32: Teilsensor
- 38: Signalpfad
- 40: Signalpfad
- 42: Signalpfad
- 44: Signalpfad
- 46: Signalpfad
- 50: Sensorsignal
- 52: Sensorsignal
- 54: Sensorsignal
- 56: Sensorsignal
- 56A: Sensorsignal
- 56B: Sensorsignal
- 56C: Sensorsignal
- 58: Sensorsignal
- 60: Sensorsignal
- 62: Sensorsignal
- 70: Referenzgas
- 72: Referenzgas
- 74: Referenzgas
- 80: Bereich
- 82: Bereich
- 82: Bereich
- 100: Vorrichtung
- 102: Signalerfassungsinstanz
- 104: Auswertesinstanz
- 106: Kommunikationsinstanz
- 110: Zählwerk
- 114: Messvolumen
- 116: Einheit
- 120: Zählereinrichtung
- 122: Zählereinrichtung
- 130: Gebäude
- 150: Zentraleinheit
- 170: Gasversorgungsleitung
- 172: Teilstrang
- 174: Teilstrang

## Patentansprüche

1. Verfahren zur Bestimmung eines Brennwerts eines methanhaltigen Brenngases, das ein oder mehrere Gasbestandteile in seiner Gaszusammensetzung aufweist, umfassend
(i) Beaufschlagen einer Sensoreinheit (10), die eine Mehrzahl von Teilsensoren (20, 22, 24, 26, 28, 30, 32) umfasst, mit dem Brenngas, wobei unterschiedliche Teilsensoren (20, 22, 24, 26, 28, 30, 32) unterschiedlich sensitiv auf denselben Gasbestandteil reagieren und/oder derselbe Teilsensor (20, 22, 24, 26, 28, 30, 32) unterschiedlich sensitiv auf unterschiedliche Konzentrationen eines jeweils gleichen Gasbestandteils in der Gaszusammensetzung reagiert;
wobei die Teilsensoren (20, 22, 24, 26, 28, 30, 32) als unterschiedlich zusammengesetzte und/oder dotierte Halbleitersensoren ausgeführt sind, die durch UV-Bestrahlung und/oder IR-Bestrahlung und/oder eine Mikroheizung unterschiedlich aktiviert werden und durch die Anwesenheit reduzierender oder oxidierender Gasbestandteile eine messbare Widerstandsänderung erfahren,
(ii) Erfassen der jeweiligen Widerstandsänderung als Sensorsignal (50, 52, 54, 56, 58, 60, 62, 56A, 56B, 56C), das von der Gaszusammensetzung und/oder der Konzentration von einem oder mehreren Gasbestandteilen abhängig ist;
(iii) Generieren eines Ist-Gasbeschaffenheitsmusters, das jeden Teilsensor (20, 22, 24, 26, 28, 30, 32) und dessen jeweiliges Sensorsignal (50, 52, 54, 56, 58, 60, 62, 56A, 56B, 56C) repräsentiert;
(iv) Bestimmen eines nächstkommenden Soll-Gasbeschaffenheitsmusters aus einer Gruppe von Soll-Gasbeschaffenheitsmustern;
(v) Extrahieren der Gaszusammensetzung und/oder der Konzentration von einem oder mehreren Gasbestandteilen aus dem nächstkommenden Soll-Gasbeschaffenheitsmuster oder mittels Interpolation aus mehreren Soll-Gasbeschaffenheitsmustern;
(vi) Bestimmen des aktuellen Brennwerts des Brenngases aus dem nächstkommenden Soll-Gasbeschaffenheitsmuster statt durch analytische Berechnung durch Vergleich des durch die Sensoreinheit (10) gelieferten Ist-Gasbeschaffenheitsmusters mit dem nächstkommenden Soll-Gasbeschaffenheitsmuster oder mittels Interpolation aus mehreren Soll-Gasbeschaffenheitsmustern.

2. Verfahren nach Anspruch 1, wobei die Soll-Gasbeschaffenheitsmuster in einer Lernphase mittels mathematischer Methoden, insbesondere mittels neuronaler Netze, generiert werden und/oder wobei das Ist-Gasbeschaffenheitsmuster mittels mathematischer Methoden, insbesondere mittels neuronaler Netze, analysiert wird.

3. Verfahren nach Anspruch 1 oder 2, wobei die Soll-Gasbeschaffenheitsmuster generiert werden, indem die Sensoreinheit (10) mit Referenzgasen mit einer vorgegebenen Konzentration an ein oder mehreren Gasbestandteilen beaufschlagt wird und den erhaltenen Sensorsignalen (50, 52, 54, 56, 58, 60, 62, 56A, 56B, 56C) als Soll-Gasbeschaffenheitsmuster ein jeweils bekannter Brennwert des Referenzgases zugeordnet wird.

4. Verfahren nach Anspruch 2 oder 3, wobei eine Schrittweite einer Konzentrationsänderung eines dem Referenzgas beigemischten Gasbestandteils in der Lernphase beim Generieren der Soll-Gasbeschaffenheitsmuster so gewählt wird, dass Brennwert-Differenzen bei der Lernphase für alle Gasbestandteile kleiner sind als eine maximal zulässige Messtoleranz.

5. Verfahren nach Anspruch 3 oder 4, wobei dem Soll-Gasbeschaffenheitsmuster ein bei der Beaufschlagung mit dem Referenzgas herrschender Druck und/oder eine Temperatur zugeordnet wird.

6. Verfahren nach einem der vorhergehenden Ansprüche, wobei zumindest der aktuelle Brennwert in einem festen Zeitintervall und/oder ereignisbezogen, insbesondere bei einer Veränderung des Brennwerts um mindestens 0,5%, vorzugsweise um mindestens 1%, an eine Zentraleinheit (150) weitergeleitet wird.

7. Verfahren nach einem der vorhergehenden Ansprüche, wobei die jeweiligen Sensorsignale (50, 52, 54, 56, 58, 60, 62, 56A, 56B, 56C) in sauerstoffarmer oder sauerstofffreier Umgebung erfasst werden.

8. Verfahren nach einem der vorhergehenden Ansprüche, wobei die Konzentration von wenigstens einem Gasbestandteil mindestens im Promillebereich, insbesondere im Prozentbereich, erfasst wird.

9. Verfahren nach einem der vorhergehenden Ansprüche, wobei die Sensoreinheit (10) die Brennwerte für mehrere Zählereinrichtungen (120) erfasst,
oder
wobei jeweils eine Sensoreinheit (10) den Brennwert jeweils einer Zählereinrichtung (122) erfasst.

10. Verfahren nach einem der vorhergehenden Ansprüche, wobei die Teilsensoren (20, 22, 24, 26, 28, 30, 32) zum Generieren des jeweiligen Sensorsignals (50, 52, 54, 56, 58, 60, 62, 56A, 56B, 56C) thermisch und/oder durch ein Aktivierungsgas und/oder durch Bestrahlung aktiviert werden.

11. Verfahren nach einem der vorhergehenden Ansprüche, wobei Sensorsignale (50, 52, 54, 56, 58, 60, 62, 56A, 56B, 56C) der Teilsensoren (20, 22, 24, 26, 28, 30, 32) zur Erzeugung des Ist-Gasbeschaffenheitsmusters kontinuierlich oder zumindest quasikontinuierlich erfasst werden.

12. Verfahren zur Bestimmung einer Energiemenge eines Gasverbrauchs aus einem erfassten Volumenwert des Gasverbrauchs und einem Brennwert, wobei der Brennwert mit einem Verfahren nach einem der vorhergehenden Ansprüche bestimmt wird, insbesondere
wobei der Brennwert kontinuierlich oder zumindest quasikontinuierlich erfasst wird.

13. Verfahren nach Anspruch 12, wobei zumindest der aktuelle Brennwert in einem festen Zeitintervall und/oder ereignisbezogen, insbesondere bei einer Veränderung des Brennwerts um mindestens 0,5%, vorzugsweise um mindestens 1%, an eine Zentraleinheit (150) weitergeleitet wird.

14. Verfahren nach einem der Ansprüche 12 bis 13, wobei der Volumenwert des Gasverbrauchs in einem festen Zeitintervall und/oder ereignisbezogen, insbesondere bei einer Veränderung des Brennwerts um mindestens 0,5%, vorzugsweise um mindestens 1%, separat von dem aktuellen Brennwert oder zusammen mit dem aktuellen Brennwert an eine Zentraleinheit (150) weitergeleitet wird.

15. Verwendung einer Sensoreinheit (10) zur Durchführung eines Verfahrens nach einem der Ansprüche 1 bis 11 oder eines Verfahrens nach einem der Anspruch 12 bis 14, wobei Teilsensoren (20, 22, 24, 26, 28, 30, 32) der Sensoreinheit (10) als Halbleitersensoren ausgebildet sind.

16. Vorrichtung (100) zur Durchführung des Verfahrens zur Bestimmung eines Brennwerts eines methanhaltigen Brenngases nach einem der Ansprüche 1 bis 11 oder eines Verfahrens zur Bestimmung einer Energiemenge eines Gasverbrauchs nach einem der Ansprüche 12 bis 14, wobei wenigstens eine Sensoreinheit (10) mit einer Mehrzahl von Teilsensoren (20, 22, 24, 26, 28, 30, 32) vorgesehen ist, wobei unterschiedliche Teilsensoren (20, 22, 24, 26, 28, 30, 32) eingerichtet sind, unterschiedlich sensitiv auf denselben Gasbestandteil zu reagieren und/oder derselbe Teilsensor (20, 22, 24, 26, 28, 30, 32) eingerichtet ist, um auf unterschiedliche Konzentrationen eines jeweils gleichen Gasbestandteils in der Gaszusammensetzung zu reagieren;
wobei die Teilsensoren (20, 22, 24, 26, 28, 30, 32) als unterschiedlich zusammengesetzte und/oder dotierte Halbleitersensoren ausgeführt sind,
wobei eine Strahlungsquelle (16) für UV-Licht, IR-Strahlung oder eine Mikroheizung (14) vorgesehen ist, dazu eingerichtet ist die Teilsensoren (20, 22, 24, 26, 28, 30, 32) unterschiedlich aktiviert und die Bandlücke des Halbleiters variiert, sodass
die Teilsensoren (20, 22, 24, 26, 28, 30, 32) durch die Anwesenheit reduzierender oder oxidierender Gasbestandteile eine messbare Widerstandsänderung erfahren,
wobei eine Signalerfassungsinstanz (102) vorgesehen ist, die eingerichtet ist die Widerstandsänderungen als Sensorsignale (50, 52, 54, 56, 58, 60, 62, 56A, 56B, 56C) der jeweiligen Teilsensoren (20, 22, 24, 26, 28, 30, 32) zu erfassen, und wobei Teilsensoren (20, 22, 24, 26, 28, 30, 32) und Sensorsignale (50, 52, 54, 56, 58, 60, 62, 56A, 56B, 56C) in jeweiligen Ist-Gasbeschaffenheitsmustern repräsentiert sind;
wobei zur Bestimmung der Energiemenge ein Zählwerk (110) vorgesehen ist, welches eingerichtet ist, das Gasvolumen zu erfassen;
wobei eine Auswerteinstanz (104) vorgesehen ist, welche eingerichtet ist, ein nächstkommendes Soll-Gasbeschaffenheitsmuster des Ist-Gasbeschaffenheitsmusters zu bestimmen und daraus einen aktuellen Brennwert und/oder die Energiemenge zu extrahieren, wobei die Bestimmung des aktuellen Brennwerts des Brenngases aus dem nächstkommenden Soll-Gasbeschaffenheitsmuster statt durch analytische Berechnung durch Vergleich des durch die Sensoreinheit (10) gelieferten Ist-Gasbeschaffenheitsmusters mit dem nächstkommenden Soll-Gasbeschaffenheitsmuster oder mittels Interpolation aus mehreren Soll-Gasbeschaffenheitsmustern erfolgt;
wobei eine Kommunikationsinstanz (106) vorgesehen ist, die eingerichtet ist den extrahierten aktuellen Brennwert oder die Energiemenge an eine Zentraleinheit (150) zu übermitteln.

17. Vorrichtung nach Anspruch 16, wobei die Auswerteinstanz (104) und/oder die Kommunikationsinstanz (106) mit einem Zählwerk (110) für ein Brenngas-Verbrauchsvolumen gekoppelt sind.

18. Vorrichtung nach Anspruch 16 oder 17, wobei zumindest die Auswerteinstanz (104), die Kommunikationsinstanz (106) und das Zählwerk (110) in einer Zählereinrichtung (122) integriert sind, insbesondere
wobei die Zählereinrichtung (122) eine Einheit (116) aufweist, welche die Auswerteinstanz (104), die Kommunikationsinstanz (106) sowie eine Recheneinheit umfasst.

19. Vorrichtung nach einem der Ansprüche 16 bis 18, wobei eine Sensoreinheit (10) mehreren Zählereinrichtungen (120) zugeordnet ist,
oder
wobei jeweils eine Sensoreinheit (10) jeweils einer Zählereinrichtung (122) zugeordnet ist.

## Claims

1. Method of determining a calorific value of a methane-containing fuel gas including one or more gas constituents in its gas composition, comprising
(i) feeding a sensor unit (10) comprising a multitude of component sensors (20, 22, 24, 26, 28, 30, 32) with the fuel gas, wherein different component sensors (20, 22, 24, 26, 28, 30, 32) react with different sensitivity to the same gas constituent and/or the same component sensor (20, 22, 24, 26, 28, 30, 32) reacts with different sensitivity to different concentrations of a respectively identical gas constituent in the gas composition;
wherein the component sensors (20, 22, 24, 26, 28, 30, 32) are designed as differently composed and/or doped semiconductor sensors that are differently activated by UV irradiation and/or IR irradiation and/or microscale heating and are subject to a measurable change in resistance as a result of the presence of reducing or oxidizing gas constituents,
(ii) detecting the respective change in resistance as sensor signal (50, 52, 54, 56, 58, 60, 62, 56A, 56B, 56C) which is dependent on the gas composition and/or the concentration of one or more gas constituents;
(iii) generating an actual gas characteristics pattern that represents each component sensor (20, 22, 24, 26, 28, 30, 32) and the respective sensor signal (50, 52, 54, 56, 58, 60, 62, 56A, 56B, 56C) therefrom;
(iv) determining a closest target gas characteristics pattern from a group of target gas characteristics patterns;
(v) extracting the gas composition and/or the concentration of one or more gas constituents from the closest target gas characteristics pattern or by interpolation from multiple target gas characteristics patterns;
(vi) determining the current calorific value of the fuel gas from the closest target gas characteristics pattern rather than by analytical calculation by comparison of the actual gas characteristics pattern given by the sensor unit (10) with the closest target gas characteristics pattern or by interpolation from multiple target gas characteristics patterns.

2. Method according to Claim 1, wherein the target gas characteristics patterns are generated in a learning phase by means of mathematical methods, especially by means of neural networks, and/or wherein the actual gas characteristics pattern is analysed by mathematical methods, especially by means of neural networks.

3. Method according to Claim 1 or 2, wherein the target gas characteristics patterns are generated by feeding the sensor unit (10) with reference gases having a defined concentration of one or more gas constituents and assigning a respectively known calorific value of the reference gas to the sensor signals obtained (50, 52, 54, 56, 58, 60, 62, 56A, 56B, 56C) as target gas characteristics pattern.

4. Method according to Claim 2 or 3, wherein a step width in a change in concentration of a gas constituent added to the reference gas is chosen in the learning phase in the generating of the target gas characteristics patterns such that differences in calorific value in the learning phase for all gas constituents are less than a maximally permissible measurement tolerance.

5. Method according to Claim 3 or 4, wherein a pressure and/or a temperature that exists on feeding with the reference gas is assigned to the target gas characteristics pattern.

6. Method according to any of the preceding claims, wherein at least the current calorific value is passed on to a central unit (150) within a fixed time interval and/or in an event-based manner, especially in the event of a change in calorific value by at least 0.5%, preferably by at least 1%.

7. Method according to any of the preceding claims, wherein the respective sensor signals (50, 52, 54, 56, 58, 60, 62, 56A, 56B, 56C) are detected in a low-oxygen or oxygen-free environment.

8. Method according to any of the preceding claims, wherein the concentration of at least one gas constituent is detected at least in the per mille range, especially in the per cent range.

9. Method according to any of the preceding claims, wherein the sensor unit (10) detects the calorific values for multiple meter devices (120),
or
wherein one sensor unit (10) in each case detects the calorific value from each meter device (122).

10. Method according to any of the preceding claims, wherein the component sensors (20, 22, 24, 26, 28, 30, 32), for generation of the respective sensor signal (50, 52, 54, 56, 58, 60, 62, 56A, 56B, 56C), are activated thermally and/or by means of an activation gas and/or by irradiation.

11. Method according to any of the preceding claims, wherein sensor signals (50, 52, 54, 56, 58, 60, 62, 56A, 56B, 56C) from the component sensors (20, 22, 24, 26, 28, 30, 32), for creation of the actual gas characteristics pattern, are detected continuously or at least quasi-continuously.

12. Method of determining an amount of energy from a consumption of gas from a detected volume value of gas consumption and a calorific value, wherein the calorific value is determined by a method according to any of the preceding claims, especially
wherein the calorific value is detected continuously or at least quasi-continuously.

13. Method according to Claim 12, wherein at least the current calorific value is passed on to a central unit (150) within a fixed time interval and/or in an event-based manner, especially in the event of a change in calorific value by at least 0.5%, preferably by at least 1%.

14. Method according to either of Claims 12 and 13, wherein the volume value of the consumption of gas is passed on to a central unit (150) separately from the current calorific value or together with the actual calorific value within a fixed time interval and/or in an event-based manner, especially in the event of a change in calorific value by at least 0.5%, preferably by at least 1%.

15. Use of a sensor unit (10) for performance of a method according to any of Claims 1 to 11 or of a method according to any of Claims 12 to 14, wherein component sensors (20, 22, 24, 26, 28, 30, 32) of the sensor unit (10) take the form of semiconductor sensors.

16. Apparatus (100) for implementation of the method of determining a calorific value of a methane-containing fuel gas according to any of Claims 1 to 11 or of a method of determining an amount of energy in a consumption of gas according to any of Claims 12 to 14, wherein at least one sensor unit (10) with a multitude of component sensors (20, 22, 24, 26, 28, 30, 32) is provided, wherein different component sensors (20, 22, 24, 26, 28, 30, 32) are set up to react with different sensitivity to the same gas constituent and/or the same component sensor (20, 22, 24, 26, 28, 30, 32) is set up to react to different concentrations of a respectively identical gas constituent in the gas composition;
wherein the component sensors (20, 22, 24, 26, 28, 30, 32) are designed as differently composed and/or doped semiconductor sensors,
wherein a radiation source (16) for UV light or IR radiation or a microscale heater (14) is provided, set up such that it differently activates component sensors (20, 22, 24, 26, 28, 30, 32) and varies the bandgap of the semiconductor, such that the component sensors (20, 22, 24, 26, 28, 30, 32) are subject to a measurable change in resistance as a result of the presence of reducing or oxidizing gas constituents,
wherein an instance of signal detection (102) set up to detect the changes in resistance as sensor signals (50, 52, 54, 56, 58, 60, 62, 56A, 56B, 56C) from the respective component sensors (20, 22, 24, 26, 28, 30, 32) is provided, and wherein component sensors (20, 22, 24, 26, 28, 30, 32) and sensor signals (50, 52, 54, 56, 58, 60, 62, 56A, 56B, 56C) are represented in respective actual gas characteristics patterns;
wherein the amount of energy is determined by providing a meter (110) set up to detect the volume of gas;
wherein an evaluation instance (104) set up to determine a closest target gas characteristics pattern of the actual gas characteristics pattern and to extract a current calorific value and/or the amount of energy therefrom is provided, wherein the current calorific value of the fuel gas is determined from the closest target gas characteristics pattern rather than by analytical calculation via comparison of the actual gas characteristics pattern provided by the sensor unit (10) with the closest target gas characteristics pattern or by interpolation from multiple target gas characteristics patterns;
wherein a communication instance (106) set up to communicate the extracted current calorific value or the amount of energy to a central unit (150) is provided.

17. Apparatus according to Claim 16, wherein the evaluation instance (104) and/or the communication instance (106) is coupled to a meter (110) for a volume of fuel gas consumption.

18. Apparatus according to Claim 16 or 17, wherein at least the evaluation instance (104), the communication instance (106) and the meter (110) are integrated in a meter device (122), especially
wherein the meter device (122) has a unit (116) comprising the evaluation instance (104), the communication instance (106) and a computation unit.

19. Apparatus according to any of Claims 16 to18, wherein a sensor unit (10) is assigned to multiple meter devices (120),
or
wherein one sensor unit (10) is assigned in each case to each meter device (122).

## Revendications

1. Procédé de détermination d'un pouvoir calorifique d'un gaz combustible contenant du méthane qui présente un ou plusieurs composants gazeux dans sa composition gazeuse, comprenant
(i) l'alimentation d'une unité de capteur (10) qui comporte une pluralité de capteurs partiels (20, 22, 24, 26, 28, 30, 32), en gaz combustible, dans lequel différents capteurs partiels (20, 22, 24, 26, 28, 30, 32) réagissent avec des sensations différentes au même composant gazeux et/ou le même capteur partiel (20, 22, 24, 26, 28, 30, 32) réagit avec des sensations différentes à différentes concentrations d'un composant gazeux respectivement identique dans la composition gazeuse ;
dans lequel les capteurs partiels (20, 22, 24, 26, 28, 30, 32) sont réalisés comme des capteurs à semi-conducteur composés et/ou dotés différemment qui sont activés différemment par rayonnement UV et/ou rayonnement IR et/ou un microchauffage et subissent une modification de résistance mesurable en raison de la présence de composants gazeux réducteurs ou oxydants,
(ii) la détection de la modification de résistance respective comme signal de capteur (50, 52, 54, 56, 58, 60, 62, 56A, 56B, 56C) qui dépend de la composition gazeuse et/ou de la concentration d'un ou plusieurs composants gazeux ;
(iii) la génération d'un motif de nature de gaz réel qui représente chaque capteur partiel (20, 22, 24, 26, 28, 30, 32) et son signal de capteur (50, 52, 54, 56, 58, 60, 62, 56A, 56B, 56C) respectif ;
(iv) la détermination d'un motif de nature de gaz de consigne le plus proche parmi un groupe de motifs de nature de gaz de consigne ;
(v) l'extraction de la composition gazeuse et/ou de la concentration d'un ou plusieurs composants gazeux du motif de nature de gaz de consigne le plus proche ou par interpolation de plusieurs motifs de nature de gaz de consigne ;
(vi) la détermination du pouvoir calorifique actuel du gaz combustible à partir du motif de nature de gaz de consigne le plus proche plutôt que par le calcul analytique par comparaison du motif de nature de gaz réel fourni par l'unité de capteur (10) avec le motif de nature de gaz de consigne le plus proche ou par interpolation de plusieurs motifs de nature de gaz de consigne.

2. Procédé selon la revendication 1, dans lequel les motifs de nature de gaz de consigne sont générés au moyen de méthodes mathématiques, en particulier au moyen de réseaux neuronaux et/ou dans lequel le motif de nature de gaz réel est analysé au moyen de méthodes mathématiques, en particulier au moyen de réseaux neuronaux.

3. Procédé selon la revendication 1 ou 2, dans lequel les motifs de nature de gaz de consigne sont générés en ce que l'unité de capteur (10) est alimentée en gaz de référence avec une concentration prédéfinie d'un ou plusieurs composants gazeux et un pouvoir calorifique respectivement connu du gaz de référence est associé aux signaux de capteur reçus (50, 52, 54, 56, 58, 60, 62, 56A, 56B, 56C) comme motifs de nature de gaz de consigne.

4. Procédé selon la revendication 2 ou 3, dans lequel un incrément d'une modification de concentration d'un composant gazeux mélangé au gaz de référence est choisi dans la phase d'apprentissage lors de la génération des motifs de nature de gaz de consigne, de sorte que des différences de pouvoir calorifique lors de la phase d'apprentissage pour tous les composants gazeux soient inférieures à une tolérance de mesure maximale autorisée.

5. Procédé selon la revendication 3 ou 4, dans lequel une pression régnant lors de l'alimentation en gaz de référence et/ou une température est associée au motif de nature de gaz de consigne.

6. Procédé selon l'une quelconque des revendications précédentes, dans lequel au moins le pouvoir calorifique actuel est transmis dans un intervalle de temps fixe et/ou par rapport à l'événement, en particulier lors d'une modification du pouvoir calorifique d'au moins 0,5 %, de préférence d'au moins 1 %, à une unité centrale (150).

7. Procédé selon l'une quelconque des revendications précédentes, dans lequel les signaux de capteur (50, 52, 54, 56, 58, 60, 62, 56A, 56B, 56C) respectifs sont détectés dans un environnement pauvre en oxygène ou sans oxygène.

8. Procédé selon l'une quelconque des revendications précédentes, dans lequel la concentration d'au moins un composant gazeux est détectée au moins dans la plage pour mille, en particulier dans la plage pour cent.

9. Procédé selon l'une quelconque des revendications précédentes, dans lequel l'unité de capteur (10) détecte les pouvoirs calorifiques pour plusieurs dispositifs compteurs (120), ou
dans lequel respectivement une unité de capteur (10) détecte le pouvoir calorifique respectivement d'un dispositif compteur (122).

10. Procédé selon l'une quelconque des revendications précédentes, dans lequel les capteurs partiels (20, 22, 24, 26, 28, 30, 32) sont activés pour la génération du signal de capteur (50, 52, 54, 56, 58, 60, 62, 56A, 56B, 56C) respectif thermiquement et/ou par un gaz d'activation et/ou par rayonnement.

11. Procédé selon l'une quelconque des revendications précédentes, dans lequel des signaux de capteur (50, 52, 54, 56, 58, 60, 62, 56A, 56B, 56C) des capteurs partiels (20, 22, 24, 26, 28, 30, 32) sont détectés pour la génération du motif de nature de gaz réel en continu ou au moins quasiment en continu.

12. Procédé de détermination d'une quantité d'énergie d'une consommation de gaz à partir d'une valeur de volume détectée de la consommation de gaz et d'un pouvoir calorifique, dans lequel le pouvoir calorifique est déterminé avec un procédé selon l'une quelconque des revendications précédentes, en particulier
dans lequel le pouvoir calorifique est détecté en continu ou au moins quasiment en continu.

13. Procédé selon la revendication 12, dans lequel au moins le pouvoir calorifique actuel est transmis dans un intervalle de temps fixe et/ou par rapport à l'événement, en particulier lors d'une modification du pouvoir calorifique d'au moins 0,5 %, de préférence d'au moins 1 %, à une unité centrale (150).

14. Procédé selon l'une quelconque des revendications 12 à 13, dans lequel la valeur de volume de la consommation de gaz est transmise dans un intervalle de temps fixe et/ou par rapport à l'événement, en particulier lors d'une modification du pouvoir calorifique d'au moins 0,5 %, de préférence d'au moins 1 %, séparément du pouvoir calorifique actuel ou conjointement avec le pouvoir calorifique actuel à une unité centrale (150).

15. Utilisation d'une unité de capteur (10) pour la réalisation d'un procédé selon l'une quelconque des revendications 1 à 11 ou d'un procédé selon l'une quelconque des revendications 12 à 14, dans laquelle des capteurs partiels (20, 22, 24, 26, 28, 30, 32) de l'unité de capteur (10) sont réalisés comme des capteurs à semi-conducteur.

16. Dispositif (100) pour la réalisation du procédé de détermination d'un pouvoir calorifique d'un gaz combustible contenant du méthane selon l'une quelconque des revendications 1 à 11, ou d'un procédé de détermination d'une quantité d'énergie d'une consommation de gaz selon l'une quelconque des revendications 12 à 14, dans lequel au moins une unité de capteur (10) est prévue avec une pluralité de capteurs partiels (20, 22, 24, 26, 28, 30, 32), dans lequel différents capteurs partiels (20, 22, 24, 26, 28, 30, 32) sont conçus afin de réagir avec des sensibilités différentes au même composant gazeux et/ou le même capteur partiel (20, 22, 24, 26, 28, 30, 32) est conçu afin de réagir à différentes concentrations d'un composant gazeux identique respectivement dans la composition gazeuse ;
dans lequel les capteurs partiels (20, 22, 24, 26, 28, 30, 32) sont réalisés comme capteurs à semi-conducteur composés et/ou dotés différemment, dans lequel une source de rayonnement (16) est prévue pour la lumière UV ou le rayonnement IR ou un microchauffage (14) conçu afin qu'il active les capteurs partiels (20, 22, 24, 26, 28, 30, 32) différemment et varie l'écart énergétique du semi-conducteur, de sorte que les capteurs partiels (20, 22, 24, 26, 28, 30, 32) subissent une modification de résistance mesurable en raison de la présence de composants gazeux réducteurs ou oxydants,
dans lequel une instance de détection de signal (102) est prévue, laquelle est conçue afin de détecter les modifications de résistance comme signaux de capteur (50, 52, 54, 56, 58, 60, 62, 56A, 56B, 56C) des capteurs partiels (20, 22, 24, 26, 28, 30, 32) respectifs, et dans lequel des capteurs partiels (20, 22, 24, 26, 28, 30, 32) et des signaux de capteur (50, 52, 54, 56, 58, 60, 62, 56A, 56B, 56C) sont représentés dans des motifs de nature de gaz réels respectifs ;
dans lequel un compteur (110) est prévu pour la détermination de la quantité d'énergie, lequel est conçu afin de détecter le volume de gaz ;
dans lequel une instance d'évaluation (104) est prévue, laquelle est conçue afin de déterminer un motif de nature de gaz de consigne le plus proche du motif de nature de gaz réel et d'en extraire un pouvoir calorifique actuel et/ou la quantité d'énergie, dans lequel la détermination du pouvoir calorifique actuel du gaz combustible à partir du motif de nature de gaz de consigne le plus proche est effectuée plutôt que par le calcul analytique par comparaison du motif de nature de gaz réel fourni par l'unité de capteur (10) avec le motif de nature de gaz de consigne le plus proche ou par interpolation de plusieurs motifs de nature de gaz de consigne ;
dans lequel une instance de communication (106) est prévue, laquelle est conçue afin de transmettre le pouvoir calorifique actuel extrait ou la quantité d'énergie à une unité centrale (150).

17. Dispositif selon la revendication 16, dans lequel l'instance d'évaluation (104) et/ou l'instance de communication (106) est couplée à un compteur (110) pour un volume de consommation de gaz combustible.

18. Dispositif selon la revendication 16 ou 17, dans lequel au moins l'instance d'évaluation (104), l'instance de communication (106) et le compteur (110) sont intégrés dans un dispositif compteur (122), en particulier
dans lequel le dispositif compteur (122) présente une unité (116) qui comporte l'instance d'évaluation (104), l'instance de communication (106) ainsi qu'une unité de calcul.

19. Dispositif selon l'une quelconque des revendications 16 à 18, dans lequel une unité de capteur (10) est associée à plusieurs dispositifs compteurs (120), ou
dans lequel respectivement une unité de capteur (10) est associée respectivement à un dispositif compteur (122).
